# EUROPEAN PATENT APPLICATION

(11) **EP 4 692 128 A1**
(43) Date of publication of application: **11.02.2026**
(21) Application number: 24778311.1
(22) Date of filing: 01.04.2024
(51) Int. Cl.: C07K 19/00, C12N 15/62, C12N 15/79, C12N 1/21, A61P 35/00

(54) **FUSION PROTEIN, CORRESPONDING NUCLEIC ACID, IN-VITRO SYNTHESIS SYSTEM, AND PREPARATION METHOD**

(30) Priority: 31.03.2023 CN 202310340138
(71) Applicant: Kangma-Healthcode (Shanghai) Biotech Co., Ltd, Pudong New Area Shanghai 201321 (CN)
(72) Inventor: GUO, Min, Shanghai 201321 (CN); WANG, Haipeng, Shanghai 201321 (CN); YANG, Yang, Shanghai 201321 (CN); HU, Ran, Shanghai 201321 (CN); XIAO, Song, Shanghai 201321 (CN); LIU, Zhang, Shanghai 201321 (CN); YU, Xue, Shanghai 201321 (CN)
(74) Representative: Dai, Simin
(86) International application number: PCT/CN2024/085334
(87) International publication number: WO 2024/199526

(57) **Abstract**

Provided is a fusion protein, which can be produced at a low cost, in a short period, and with a high yield. Also provided are a coding nucleic acid of the fusion protein, an in-vitro synthesis system, a preparation method, and the like. The fusion protein has: an effector moiety A for killing a target cell, including a toxin molecule; a targeting vector moiety B that binds to a target site on the target cell, the targeting vector moiety being derived from an antibody or a cytokine; and a first linker L1 for linking the effector moiety A to the targeting vector moiety B, wherein the first linker L1 comprises at least 3 amino acid residues. Preferably, the linking mode of the effector moiety A, the first linker L1, and the targeting vector moiety B from the N-terminus to the C-terminus is A-L1-B or B-L1-A.

## Description

### TECHNICAL FIELD

The present disclosure belongs to the field of biotechnology, and specifically relates to a fusion protein, a corresponding nucleic acid, an *in vitro* synthesis system, and a preparation method.

### BACKGROUND

Globally, the incidence of cancer is gradually increasing, the number of patients is rising year by year, and the number of deaths due to cancer is gradually increasing annually. In traditional cancer treatment, methods such as chemotherapy and radiotherapy often cause suffering to patients due to their poor selectivity between cancer cells and normal cells. Selective immunotherapies have developed rapidly in recent years, such as targeted drugs, monoclonal antibodies, bispecific antibodies, ADCs, and immunotoxins.

Immunotoxins generally comprise a targeting vector moiety and an effector moiety, wherein the targeting vector moiety is responsible for binding immunotoxin molecules to tumor cells carrying target sites, and the effector moiety is responsible for killing the tumor cells. The targeting vector moiety of immunotoxins is often derived from the immune system, including antibodies, cytokines, and growth factors. The effector moiety consists of toxin proteins, wherein the binding domain of naturally occurring toxin proteins is removed and replaced with the targeting vector moiety, enabling the toxin protein to acquire the ability to target tumor cells. These toxin proteins may be derived from bacteria, fungi, plants, or even animals. Commonly used bacterial toxin proteins include Cholerae toxin (ChxA), Shiga toxin, Pseudomonas exotoxin A (PE), and Diphtheria toxin (DT).

Immunotoxins have undergone three generations of development:

The first generation of immunotoxins used intact monoclonal antibody molecules. Although the specificity and stability of the antibodies were not problematic, the molecules were too large to effectively penetrate tissues and reach target sites, and their high immunogenicity was prone to induce immune responses. Therefore, this approach is no longer in use.

The second generation of immunotoxins used Fab fragments instead of intact antibody molecules, which solved the problem of excessively large molecules, but still relied on chemical conjugation to link the toxin proteins to the antibodies. Chemical conjugation requires the addition of chemical reagents, resulting in low conjugation efficiency, high production costs, and poor product homogeneity due to the numerous possible conjugation sites on the proteins. Moreover, chemical conjugation bonds are prone to breakage, leading to toxic leakage and significant toxic side effects. Additionally, the naked antibodies produced upon degradation may block antigens, resulting in poor therapeutic efficacy.

The third generation of immunotoxins are expressed by recombinant genetic engineering, in which the genes encoding targeting polypeptides are fused with the genes encoding toxin polypeptides and expressed in an appropriate expression system. Compared to immunotoxins produced by chemical conjugation, recombinant immunotoxins show greatly improved product homogeneity and stability, and enable large-scale production of immunotoxins. However, current production primarily relies on cellular expression, which still has some limitations.

For example, *Escherichia coli* expression systems are commonly used to express single-chain antibody immunotoxins. The targeting moiety of immunotoxins often fails to fold properly in the *E. coli* expression system, resulting in inclusion bodies. The refolding of inclusion bodies is a highly complex process, with the refolding efficiency of proteins typically around 20%. Secretion in eukaryotic systems can overcome some of the defects of the *E. coli* expression system, but it involves long fermentation cycles and high costs, making it difficult to compete in an industrial scale.

Furthermore, in the third generation of immunotoxins, the Fv fragment of antibodies is commonly used for construction, as it is the smallest unit containing a complete antigen-binding site, has a small molecular size, and is easily expressed in cellular systems. However, the Fv structure is unstable *in vivo* and prone to dissociation due to the inability to form a covalent bond between VH and VL.

### SUMMARY

The present disclosure provides a suitable fusion protein, which can be produced at a low cost, in a short period, and with a high yield, as well as a corresponding nucleic acid, an *in vitro* synthesis system, and a preparation method to overcome the aforementioned defects of existing immunotoxins.

For this purpose, the present disclosure provides the following technical solutions:

The present disclosure provides a fusion protein, comprising: an effector moiety A for exerting killing effect on a target cell, including a toxin molecule; a targeting vector moiety B that binds to a target site on the target cell, the targeting vector moiety being derived from an antibody or a cytokine; and a first linker L1 for linking the effector moiety A to the targeting vector moiety B, wherein the first linker L1 comprises at least 3 amino acid residues, preferably 3 to 20 amino acid residues, and more preferably 3 to 10 amino acid residues; further preferably, the effector moiety A, the first linker L1, and the targeting vector moiety B are connected in an orientation of A-L1-B from the N-terminus to the C-terminus or in an orientation of B-L1-A from the N-terminus to the C-terminus.

The fusion protein provided by the present disclosure also has the following characteristics: the toxin molecule is any one of the following toxic polypeptides: (a) a plant-derived toxin; (b) a bacterial-derived toxin; (c) a fungal-derived toxin; (d) a human-derived protein toxin; (e) a functional fragment of any one of (a) to (d); (f) a functional fragment derived from any one of (a) to (d) or a variant of the functional fragment; preferably, the toxin molecule is derived from: Diphtheria toxin, Pseudomonas exotoxin A, or Cholerae toxin.

The fusion protein provided by the present disclosure also has the following characteristics: the structure of the toxin molecule comprised in the effector moiety is any one of the following:
(1) a translocation domain DT and a catalytic domain DA of Diphtheria toxin, preferably connected in an orientation of DA-DT from the N-terminus to the C-terminus;
(2) the catalytic domain DA of Diphtheria toxin;
(3) a translocation domain PT and a catalytic domain PA of Pseudomonas exotoxin A, preferably connected in an orientation of PT-PA from the N-terminus to the C-terminus;
(4) the catalytic domain PA of Pseudomonas exotoxin A;
(5) a translocation domain CT and a catalytic domain CA of Cholerae toxin, preferably connected in an orientation of CT-CA from the N-terminus to the C-terminus;
(6) the catalytic domain CA of Cholerae toxin.

The fusion protein provided by the present disclosure also has the following characteristics: the toxin molecule has an amino acid sequence shown in any one of SEQ ID NOs: 1 to 9, or has an amino acid sequence with at least 80%, 85%, 90%, 95%, or 99% sequence identity to any one of SEQ ID NOs: 1 to 9.

The fusion protein provided by the present disclosure also has the following characteristics: the targeting vector moiety is an antibody, wherein the antibody is any one or more of a monoclonal antibody, a chimeric antibody, a reshaped antibody, a small molecule antibody, a multispecific antibody, and a multivalent antibody;
preferably, the antibody is any one or more of a small molecule antibody or a multivalent antibody;
more preferably:
the small molecule antibody is any one or more of Fv, Fab, ScFv, ScFab, ScIgG, dsFv, a nanobody, and a single-domain antibody;
the multivalent antibody is a tandem antibody formed by at least two single-chain antibodies in tandem and an antibody polymeric fusion protein formed by a small molecule antibody and a polymeric protein; further, the antibody polymeric fusion protein is a dimer or a tetramer.

The fusion protein provided by the present disclosure also has the following characteristics: the antibody is ScFab, ScFv, ScIgG, a nanobody, two ScFabs in tandem, two ScFvs in tandem, two nanobodies in tandem, a fusion of ScFab with an antibody fragment CH3, a fusion of ScFv with an antibody fragment CH3, two nanobodies fused in tandem via CH3 (e.g., VHH1-L3-CH3-L3-VHH2), a fusion of ScFab with Tamavidins2 protein, or a fusion of ScFv with Tamavidins2 protein.

The fusion protein provided by the present disclosure also has the following characteristics: the structure of ScFab satisfies the following: the light chain portion and the heavy chain portion of ScFab are connected in an orientation from the N-terminus to the C-terminus, or the heavy chain portion and the light chain portion of ScFab are connected in an orientation from the N-terminus to the C-terminus; ScFab and the antibody fragment CH3 are connected in an orientation from the N-terminus to the C-terminus or from the C-terminus to the N-terminus; the heavy chain portion and the light chain portion of ScFv are connected in an orientation from the N-terminus to the C-terminus; the fusion of ScFab with Tamavidins2 protein is connected in an orientation from the N-terminus to the C-terminus as Tamavidins2 protein to ScFab; the fusion of ScFv with Tamavidins2 protein is connected in an orientation from the N-terminus to the C-terminus as Tamavidins2 protein to ScFv.

The fusion protein provided by the present disclosure also has the following characteristics: the light chain portion and the heavy chain portion of each of ScFab, ScFv, ScFv, and ScIgG are connected via a second linker L2, wherein the second linker L2 comprises at least 15 amino acid residues, preferably 15 to 180, 15 to 120, 15 to 90, 15 to 65, or 20 to 60 amino acid residues.

The fusion protein provided by the present disclosure also has the following characteristics: the second linker L2 comprises any one or more of predominant amino acid residues of G, S, T, and A, and the number of the predominant amino acid residues accounts for at least more than 50%, 60%, 70%, 80%, or 90% of the total number of amino acids in the second linker; more preferably, the second linker L2 comprises any one or more of G and S among the predominant amino acid residues, and the total number of the predominant amino acid residues accounts for at least more than 40%, or more than 45%, or more than 60%, or more than 70%, or more than 80% of the total number of amino acid residues in the second linker;
further, the second linker L2 has an amino acid sequence shown in any one of SEQ ID NOs: 10, 12, 13, 14, 17, 28, 45, and 47, or has an amino acid sequence with at least 80%, 85%, 90%, 95%, or 99% sequence identity to any one of SEQ ID NOs: 10, 12, 13, 14, 17, 28, 45, and 47.

The fusion protein provided by the present disclosure also has the following characteristics: two single-chain antibodies, or a small molecule antibody and a polymeric protein, are connected via a third linker L3, wherein the third linker L3 is absent or comprises at least 3 amino acid residues; preferably, the third linker L3 comprises 5 to 20 amino acid residues; preferably, the third linker L3 comprises 8 to 20 amino acid residues, or 8 to 15 amino acid residues, or 8 to 12 amino acid residues; more preferably, the third linker L3 comprises two predominant amino acid residues, G and S, and the number of the predominant amino acid residues accounts for more than 40% of the total number of amino acid residues in the third linker;
for example, the third linker L3 has an amino acid sequence shown in any one of SEQ ID NOs: 10, 14, 18, 20 to 30, 44, and 48 to 51, or has an amino acid sequence with at least 80%, 85%, 90%, 95%, or 99% sequence identity to any one of SEQ ID NOs: 10, 14, 18, 20 to 30, 44, and 48 to 51.

The fusion protein provided by the present disclosure also has the following characteristics: the antibody is an antibody that specifically binds to any one or more of Her2 and CD22.

The fusion protein provided by the present disclosure also has the following characteristics: the antibody is derived from IgM, IgG, IgA, IgD, and IgE; preferably, the antibody is derived from Trastuzumab, Rituximab, Bevacizumab, Adalimumab, Pembrolizumab, Ustekinumab, Nivolumab, Ocrelizumab, Palivizumab, Infliximab, Omalizumab, Envafolimab, Atlizumab, Atezolizumab, or Cetuximab, or variants thereof.

The fusion protein provided by the present disclosure also has the following characteristics:
for Fab or ScFab:
the light chain portion constituting the Fab or ScFab has an amino acid sequence shown in SEQ ID NO: 32, or has an amino acid sequence with at least 80%, 85%, 90%, 95%, or 99% sequence identity to SEQ ID NO: 32; or
the variable domain of the heavy chain portion constituting the Fab or ScFab has an amino acid sequence shown in SEQ ID NO: 33 or 55, or has an amino acid sequence with at least 80%, 85%, 90%, 95%, or 99% sequence identity to SEQ ID NO: 33 or 55; or
the first constant domain CH1 of the heavy chain portion constituting the Fab or ScFab has an amino acid sequence shown in SEQ ID NO: 34 or 57, or has an amino acid sequence with at least 80%, 85%, 90%, 95%, or 99% sequence identity to SEQ ID NO: 34 or 57;
for Fv, dsFv, or ScFv:
the variable domain of the light chain portion constituting the Fv, dsFv, or ScFv has an amino acid sequence shown in SEQ ID NO: 35 or 54, or has an amino acid sequence with at least 80%, 85%, 90%, 95%, or 99% sequence identity to SEQ ID NO: 35 or 54; or
the variable domain of the heavy chain portion constituting the Fv, dsFv, or ScFv has an amino acid sequence shown in SEQ ID NO: 33 or 55, or has an amino acid sequence with at least 80%, 85%, 90%, 95%, or 99% sequence identity to SEQ ID NO: 33 or 55;
for ScIgG:
the light chain portion constituting the ScIgG has an amino acid sequence shown in SEQ ID NO: 32 or 56, or has an amino acid sequence with at least 80%, 85%, 90%, 95%, or 99% sequence identity to SEQ ID NO: 32 or 56; or
the heavy chain portion constituting the ScIgG has an amino acid sequence shown in SEQ ID NO: 36 or 17, or has an amino acid sequence with at least 80%, 85%, 90%, 95%, or 99% sequence identity to SEQ ID NO: 36 or 17;
for the antibody fragment CH3 involved, the antibody fragment CH3 has the amino acid sequence shown in SEQ ID NO: 37, or has an amino acid sequence with at least 80%, 85%, 90%, 95%, or 99% sequence identity to SEQ ID NO: 37;
for the Tamavidins2 protein involved, the Tamavidins2 protein has the amino acid sequence shown in SEQ ID NO: 38, or has an amino acid sequence with at least 80%, 85%, 90%, 95%, or 99% sequence identity to SEQ ID NO: 38;
for the nanobody involved, the nanobody has an amino acid sequence shown in SEQ ID NO: 52 or 53, or has an amino acid sequence with at least 80%, 85%, 90%, 95%, or 99% sequence identity to SEQ ID NO: 52 or 53;
the activity of the fusion protein against target cells, particularly the IC50, is comparable to that of commercially available DS-8201a or T-DM1.

The fusion protein provided by the present disclosure also has the following characteristics: the fusion protein is suitable for cell-free *in vitro* synthesis; preferably, the cell-free *in vitro* synthesis involves: in a cell-free *in vitro* synthesis system, using a coding nucleic acid of the fusion protein as a DNA template or RNA template, incubating under certain conditions for a certain period of time to obtain the fusion protein.

The fusion protein provided by the present disclosure also has the following characteristics: the cell-free *in vitro* synthesis system comprises a cell extract, wherein the cell extract is derived from a yeast strain; further, the yeast strain is *Saccharomyces cerevisiae*, *Kluyveromyces,* or a combination thereof; in another preferred example, the *Kluyveromyces* is *Kluyveromyces lactis*, *Kluyveromyces marxianus*, *Kluyveromyces dobzhanskii*, or a combination thereof.

The fusion protein provided by the present disclosure also has the following characteristics: the yeast strain is genetically engineered to be toxin-resistant.

The fusion protein provided by the present disclosure also has the following characteristics: the first linker L1 has an amino acid sequence shown in any one of SEQ ID NOs: 10, 11, 15, 16, 18 to 20, 31, and 43, or has an amino acid sequence with at least 80%, 85%, 90%, 95%, or 99% sequence identity to any one of SEQ ID NOs: 10, 11, 15, 16, 18 to 20, 31, and 43.

The present disclosure also provides an isolated nucleic acid, wherein the nucleic acid encodes the fusion protein described above.

The present disclosure also provides a vector, wherein the vector comprises the nucleic acid described above.

The present disclosure also provides a host cell, wherein the host cell comprises the nucleic acid or vector described above; preferably, the host cell is a yeast cell; further, the yeast cell is *Saccharomyces cerevisiae*, *Kluyveromyces*, or a combination thereof; in another preferred example, the *Kluyveromyces* is *Kluyveromyces lactis*, *Kluyveromyces marxianus*, *Kluyveromyces dobzhanskii*, or a combination thereof.

The present disclosure also provides an *in vitro* cell-free protein synthesis system, wherein
an mRNA or DNA encoding the fusion protein described above is used as a template to synthesize the fusion protein.

The *in vitro* cell-free protein synthesis system provided by the present disclosure also has the following characteristics, comprising any one or more of the following:
(1) an mRNA or DNA template;
(2) a cell extract, wherein the cell extract is a yeast cell extract; further, the cell extract is derived from a strain selected from the group consisting of *Saccharomyces cerevisiae*, *Pichia pastoris*, and *Kluyveromyces,* or a combination thereof; preferably, the yeast cell extract is derived from *Kluyveromyces,* more preferably *Kluyveromyces lactis*;
(3) any one or more of the following components:
   an amino acid mixture, dNTP, RNA polymerase, DNA polymerase, an energy supply system, polyethylene glycol, and an aqueous solvent.

The *in vitro* cell-free protein synthesis system provided by the present disclosure also has the following characteristics:
the strain is genetically engineered to be toxin-resistant.

The present disclosure also provides an *in vitro* cell-free synthesis method for the fusion protein described above, wherein the method comprises: in a reaction system comprising the *in vitro* cell-free protein synthesis system described above, performing an *in vitro* synthesis reaction using an mRNA or DNA encoding the fusion protein described above as a template to obtain the fusion protein; preferably, the DNA template and other components involved in the *in vitro* synthesis reaction have a volume ratio of 1:10 to 1:50, or 1:20 to 1:40, or 1:25 to 1:35, or 1:30.

The *in vitro* cell-free synthesis method provided by the present disclosure also has the following characteristics: the *in vitro* cell-free protein synthesis system comprises a yeast cell extract, an amino acid mixture, dNTP, RNA polymerase, DNA polymerase, an energy supply system, polyethylene glycol, and an aqueous solvent; further, the yeast cell extract is derived from a strain selected from the group consisting of *Saccharomyces cerevisiae*, *Pichia pastoris*, and *Kluyveromyces,* or a combination thereof; preferably, the yeast cell extract is derived from *Kluyveromyces,* more preferably *Kluyveromyces lactis,* further, the strain is genetically engineered to be toxin-resistant.

The *in vitro* cell-free synthesis method provided by the present disclosure also has the following characteristics: the yeast cell extract accounts for 50 to 80% of the total volume of the *in vitro* cell-free protein synthesis system.

The present disclosure also provides a use of the nucleic acid described above, the vector described above, the host cell described above, or the *in vitro cell-free* protein synthesis system described above in the manufacture of the fusion protein described above.

The present disclosure also provides a use of the fusion protein described above in disease treatment, or a medicament comprising the fusion protein described above as an active ingredient for treating a disease, preferably for use in anti-tumor therapy, anti-transplant rejection, and treatment of an autoimmune disease.

The use described above provided by the present disclosure also has the following characteristics: the disease treatment is a treatment targeting any one or more of HER2 and CD22.

The present disclosure also provides a use of the nucleic acid described above, the vector described above, the host cell described above, or the fusion protein described above in the manufacture of a medicament for use in the disease treatment described above.

### Effects and advantages

The fusion protein, the corresponding nucleic acid, the *in vitro* synthesis system, and the preparation method provided by the present disclosure have been developed through extensive and in-depth research. For the first time, a fusion protein suitable for high-yield *in vitro* synthesis and production has been discovered through extensive screening and exploration. The fusion protein of the present disclosure is formed by fusing an effector moiety and a targeting vector moiety via a specific first linker, exhibiting high yield while maintaining activity comparable to that of commercially available products. Thus, the fusion protein of the present disclosure can potentially replace existing immunotoxins in terms of activity.

Moreover, the fusion protein of the present disclosure can be synthesized in an *in vitro* protein synthesis system. Compared to existing cell-based expression methods for synthesizing immunotoxins, it has a lower production cost and a shorter production cycle, and is suitable for large-scale production and promotion.

In particular, compared to current immunotoxin structures primarily based on Fv fragments, the fusion protein provided by the present disclosure is successfully expressed in a structure in which a Fab fragment serves as the targeting vector moiety B, while maintaining activity comparable to that of commercially available products.

### BRIEF DESCRIPTION OF THE DRAWINGS

FIG. 1 shows the cytotoxicity assay results of four fusion proteins in the examples;
FIGs. 2 to 4 show the cytotoxicity assays of several fusion proteins with different molecule IDs against the HER2-overexpressing breast cancer cell line SK-BR-3 in Example 4, with a commercially available ADC (RC48) as a control;
FIGs. 5 to 7 show the cytotoxicity assays of several expressed fusion proteins against the Her2-overexpressing human gastric cancer cell line NCI-N87 in Example 4, in which cells were seeded into a 96-well plate at a density of 1000, 3000, and 5000 cells per well, respectively, with the results shown in FIG. 5 (3000 cells/well), FIG. 6 (5000 cells/well), and FIG. 7 (1000 cells/well), respectively;
FIG. 8 shows changes in tumor volume after intratumoral injection in Example 5;
FIG. 9 shows tumor weight after intratumoral injection in Example 5;
FIG. 10 shows changes in tumor volume after intravenous injection via tail vein in Example 5;
FIG. 119 shows tumor weight after intravenous injection via tail vein in Example 5.

### DETAILED DESCRIPTION OF THE PREFERRED EMBODIMENT

The specific embodiments of the present disclosure are described below with reference to the accompanying drawings. For the specific methods or materials used in the examples, those skilled in the art may make conventional replacement selections based on the technical ideas of the present disclosure and according to existing technologies, and are not limited to the specific descriptions in the examples of the present disclosure.

Unless otherwise specified, the methods used in the examples are conventional methods. Unless otherwise specified, the materials and reagents used are commercially available.

The definitions of terms used herein are intended to incorporate the definitions commonly accepted in the field of biotechnology for each term. Where appropriate, illustrative examples are provided. Unless otherwise limited in specific circumstances individually or as part of a broader group, these definitions apply to the terms used throughout the present specification.

As used herein, the term "variant" refers to a polypeptide or polynucleotide sequence that differs from a reference polypeptide or polynucleotide sequence but retains its essential properties. Typically, variants are highly similar to the reference polypeptide or polynucleotide sequence overall and are identical in many regions.

For example, the variant may comprise an amino acid sequence having at least one conservative amino acid substitution relative to the parent polypeptide sequence; or, the variant may comprise an amino acid sequence having at least one non-conservative amino acid substitution relative to the parent polypeptide sequence, in which case the non-conservative amino acid substitution preferably does not interfere with or inhibit the biological activity of the functional variant, and the non-conservative amino acid substitution may enhance the biological activity of the variant, such that the biological activity of the variant is increased compared to that of the parent polypeptide.

When a reference polypeptide or polynucleotide sequence is used, the term "percent sequence identity" refers to a comparison between polynucleotides or polypeptides and is determined by comparing two optimally aligned sequences within a comparison window, wherein the polynucleotide or polypeptide sequence within the comparison window may contain additions or deletions (*i.e.*, gaps) compared to the reference sequence (which does not contain additions or deletions) for optimal alignment of the two sequences. The percentage is calculated by determining the number of positions at which identical nucleic acid bases or amino acid residues are present in both sequences to yield the number of matching positions, dividing the number of matching positions by the total number of positions in the longer sequence within the comparison window, and multiplying the result by 100 to obtain the percent sequence identity. Identity is assessed using any of a variety of sequence comparison algorithms and programs known in the art. Such algorithms and programs include, but are by no means limited to, TBLASTN, BLASTP, FASTA, TFASTA, and CLUSTALW. In certain embodiments, protein and nucleic acid sequence identity is assessed using the Basic Local Alignment Search Tool ("BLAST"), which is well known in the art (see, *e.g.*, Karlin and Altschul, 1990, Proc. Natl. Acad. Sci. USA 87: 2267-2268; Altschul et al., 1990, J. Mol. Biol. 215: 403-410; Altschul et al., 1993, Nature Genetics 3: 266-272; Altschul et al., 1997, Nuc. Acids Res. 25: 3389-3402).

As used herein, the term "cytokine" is interpreted as a class of small molecule proteins with a wide range of biological activities that are synthesized and secreted by immune cells (*e.g.*, monocytes, macrophages, T cells, B cells, NK cells) and certain non-immune cells (*e.g.*, endothelial cells, epidermal cells, fibroblasts) upon stimulation. Cytokines generally regulate cell growth, differentiation, and effector functions by binding to corresponding receptors, thereby modulating immune responses. Cytokines (CK) are low molecular weight soluble proteins induced by immunogens, mitogens, or other stimulants in a variety of cells, possessing diverse functions such as regulation of innate and adaptive immunity, hematopoiesis, cell growth, APSC pluripotent cells, and repair of damaged tissues. Cytokines can be classified into interleukins (IL), interferons, tumor necrosis factors (TNF), colony-stimulating factors (CSF), chemokines, growth factors (GF), and others.

As used herein, the term "antibody" is used in the broadest sense to refer to any immunoglobulin (Ig) molecule comprising two heavy chains and two light chains, and any antibody fragment, mutant, variant, or derivative thereof, provided that it exhibits the desired biological activity (*e.g*., epitope binding activity).

Antibodies include, but are not limited to, polyclonal antibodies, monoclonal antibodies, chimeric antibodies, fully human antibodies, domain antibodies, single-chain antibodies, Fab and F(ab')2 fragments, scFv, and Fab expression libraries.

A full antibody is a structure comprising two full-length light chains and two full-length heavy chains, with each light chain (L) connected to a heavy chain (H) via a disulfide bond. Full antibodies include IgA, IgD, IgE, IgM, and IgG, wherein IgG further comprises subclasses including IgG1, IgG2, IgG3, and IgG4. Furthermore, in humans, the light chain may be a κ chain or a λ chain.

The variable regions of the heavy and light chains are referred to as VH and VL, respectively. Recognition and binding to a target is the primary function of the N-terminal variable ("V") regions of the heavy ("H") and light ("L") chains.

The constant regions of the heavy and light chains are referred to as CH and CL, respectively. The CH domain varies in length. The constant region of the heavy chains of IgG, IgA, and IgD comprises three domains: CH1, CH2, and CH3, while the constant region of the heavy chains of IgM and IgE comprises four domains: CH1, CH2, CH3, and CH4.

The term "antibody fragment" refers to a portion of a full-length antibody, preferably the antigen-binding or variable region of the full-length antibody. Examples of antibody fragments include Fab, Fab', F(ab')2, and Fv fragments; diabodies (Db); tandem diabodies (taDb); linear antibodies (*e.g.*, U.S. Patent No. 5,641,870, Example 2; Zapata et al., Protein Eng. 8(10): 1057-1062 (1995)); single-arm antibodies, single variable domain antibodies, minibodies, single-chain antibody molecules; and multispecific antibodies formed from antibody fragments (*e.g*., including, but not limited to, Db-Fc, taDb-Fc, taDb-CH3, and (scFv)4-Fc).

As used herein, the term "monoclonal antibody (mAb)" refers to an antibody produced by a single hybridoma cell line and specific for a single antigenic epitope.

Chimeric antibodies, such as human-mouse chimeric antibodies (chimeric Ab), retain the specificity and affinity of murine monoclonal antibodies and significantly reduce their immunogenicity in humans, while also allowing for subclass switching, thereby producing antibody molecules that share the same specificity but can mediate different effector functions.

Reshaping antibodies (reshaping Ab) or humanized antibodies (humanized Ab) refer to antibodies produced by genetic engineering techniques in which the amino acid sequences of the complementary determining region (CDR) in the variable region (V) of a human antibody are replaced with the CDR sequences of a murine monoclonal antibody, resulting in a reconstructed humanized antibody that retains both the specificity and affinity of the murine monoclonal antibody.

As used herein, the term "small molecule antibody" refers to, for example, fragment of antigen-binding (Fab), fragment of variable (Fv), single-chain fragment of variable (ScFv), disulfide-stabilized Fv (dsFv), single-chain Fab (ScFab), and ScIgG.

A "Fab" fragment is an antigen-binding fragment produced by papain digestion of an antibody, consisting of the entire light chain, the variable domain of the heavy chain (VH), and the first constant domain (CH1) of one heavy chain. Papain digestion of an antibody produces two identical Fab fragments. Pepsin digestion of an antibody produces a single large F(ab')2 fragment, which roughly corresponds to two Fab fragments linked via a disulfide bond with bivalent antigen-binding activity and is still capable of cross-linking antigen. A Fab' fragment differs from a Fab fragment in that it contains additional residues at the C-terminus of the CH1 domain, including one or more cysteine residues derived from the hinge region of the antibody. As used herein, Fab'-SH refers to a Fab' fragment in which the cysteine residue(s) of the constant domain contain a free sulfhydryl group. A F(ab')2 fragment is essentially a dimer of Fab' fragments linked via cysteine residues in the hinge region.

"Fv" consists of a dimer of the variable domains of one heavy chain and one light chain in tight, non-covalent association. The folding of these two domains generates six hypervariable loops (three loops each from the heavy and light chains), which provide amino acid residues for antigen binding and confer antigen-binding specificity to the antibody. As used herein, the term "single-chain" is used in the broadest sense to refer to a single polypeptide chain formed by linking different fragments, such as two antibody fragments, with or without a linker between them.

A disulfide-stabilized Fv (dsFv) is formed by introducing a disulfide bond between the heavy chain variable region and the light chain variable region, while a single-chain Fv (scFv) is typically formed by covalently linking the heavy chain variable region and the light chain variable region through a peptide linker.

A single-chain Fab (ScFab) was reported in 2007 by Stefan Dubel in *BMC Biotechnology.* Compared to ScFv, ScFab exhibits better stability while retaining the affinity of the parent antibody. ScFab is a single polypeptide chain consisting of the entire light chain, the variable domain of the heavy chain (VH), and the first constant domain (CH1) of one heavy chain.

"ScIgG" or "single-chain cleaved IgG" refers to any immunoglobulin G molecule with a heterodimeric structure comprising two heavy chains and two light chains, in which one heavy chain has undergone proteolytic cleavage within a single heavy chain, while the other heavy chain remains intact.

A single-domain antibody contains only the heavy chain variable region and has a structure smaller than the Fv subunit, which is a molecule with antigen-binding activity. Compared to full-length antibodies, single-domain antibodies still possess equivalent antigen-binding capacity and stability.

A nanobody refers to the VHH structure that can be independently cloned and expressed, which exhibits structural stability and antigen-binding activity comparable to that of the parent heavy chain antibody, and is the smallest unit known to bind to a target antigen. The VHH crystal has a size of 2.5 nm in width and 4 nm in length, with a molecular weight of only 15 KDa, which is also referred to as a nanobody (Nb).

As used herein, the multispecific antibody refers to an antibody with the ability to specifically bind two or more different targets on the same or different antigens.

As used herein, the multivalent antibody refers to an antibody structure that contains multiple domains capable of binding to the same target, such as two identical ScFv fragments in tandem.

In each antibody structure described herein, sequences derived from the light chain (L) of a full-length antibody is collectively referred to as the light chain portion, and sequences derived from the heavy chain (H) of a full-length antibody is collectively referred to as the heavy chain portion, for example as follows.

For Fab or ScFab fragments, the light chain portion is the light chain (L) of the full-length antibody; the heavy chain portion comprises the variable domain (VH) of the heavy chain (H) of the full-length antibody and the first constant domain (CH1) of one heavy chain (H). It should be noted that the term "derived from" as used herein refers to originating from, and is not limited to the exact original sequence of the parent antibody. That is, the respective chains or domains that constitute the structure may be identical in sequence to the corresponding chain or domain of the parent antibody, or may be variants of the corresponding sequence of the parent antibody. For example, for the variable domain (VH) derived from the heavy chain (H), the amino acid sequence of the variable domain in Fab or ScFab may be the identical sequence of the variable domain of the heavy chain (H) of the parent antibody, or may be a variant thereof. The similar interpretation applies analogously to other structural elements described herein.

For ScIgG, the light chain portion is a full-length single light chain (L), and the heavy chain portion is a full-length single heavy chain (H).

For Fv, dsFv, and ScFv fragments, the light chain portion is the variable region (VL) of the full-length light chain (L); the heavy chain portion is the variable domain (VH) of the full-length heavy chain (H).

For a full-length antibody, the entire light chain (L) is also referred to as the light chain portion, and the entire heavy chain (H) is also referred to as the heavy chain portion.

As used herein, the term "activity" of the fusion protein refers to, for example, affinity values such as EC50 and/or IC50.

The term *"in vitro* cell-free protein synthesis system" in the present disclosure has the same meaning as expressions such as *"in vitro* expression system", *"in vitro* protein synthesis system", *"in vitro* protein synthesis reaction system", and "cell-free protein synthesis system", which may be described as a protein *in vitro* synthesis system, an *in vitro* protein synthesis system, a cell-free system, a cell-free protein synthesis system, a cell-free *in vitro* protein synthesis system, an *in vitro* cell-free protein synthesis system, an *in vitro* cell-free synthesis system, a CFS system (cell-free system), and a CFPS system (cell-free protein synthesis system). These terms also encompass *in vitro* translation system and *in vitro* transcription and translation system (IVTT system).

The term *"in vitro* protein synthesis reaction" refers to the synthesis of proteins in an *in vitro* cell-free synthesis system, which at least includes the translation process. The term includes, but is not limited to, an IVTT reaction (*in vitro* transcription and translation reaction). In the present disclosure, the IVTT reaction is preferred. The IVTT reaction, corresponding to an IVTT system, is a process of transcribing and translating DNA into protein *in vitro.* Accordingly, such *in vitro* protein synthesis systems are also referred to as the D2P system, D-to-P system, D_to_P system, or DNA-to-Protein system. The corresponding *in vitro* protein synthesis methods are also referred to as the D2P method, D-to-P method, D_to_P method, or DNA-to-Protein method.

In the *in vitro cell-free* synthesis method of the present disclosure, the *in vitro* protein synthesis systems, templates, plasmids, target proteins, *in vitro* protein synthesis reactions (incubation reactions), various preparation methods, various detection methods as technical elements of the present disclosure may also be independently selected from the suitable embodiments or implementation methods disclosed in the following references, including, but not limited to, CN111484998A, CN106978349A, CN108535489A, CN108690139A, CN108949801A, CN108642076A, CN109022478A, CN109423496A, CN109423497A, CN109423509A, CN109837293A, CN109971783A, CN109988801A, CN109971775A, CN110093284A, CN110408635A, CN110408636A, CN110551745A, CN110551700A, CN110551785A, CN110819647A, CN110845622A, CN110938649A, and CN110964736A. Unless conflicting with the purpose of the present disclosure, these references and their cited references are incorporated by reference in their entirety and for all purposes.

### Fusion protein

The fusion protein provided by the present disclosure is also an immunotoxin, and may also be referred to as a fusion toxin protein (FTP), comprising: an effector moiety A, a targeting vector moiety B, and a first linker L1. The connection order of the effector moiety A, the targeting vector moiety B, and the first linker L1 is not particularly limited. In one example, the effector moiety A, the first linker L1, and the targeting vector moiety B are connected in an orientation of A-L1-B from the N-terminus to the C-terminus.

The effector moiety A is used to kill a target cell, including a toxin molecule.

The targeting vector moiety B binds to a target site on the target cell to specifically target the immunotoxin to the desired location, specifically referring to any amino acid, peptide, polypeptide, or protein with specific binding affinity for a receptor or antigen on the target cell. As used herein, the targeting vector moiety is primarily derived from an antibody or a cytokine.

The first linker L1 connects the effector moiety A and the targeting vector moiety B. In one example, the first linker L1 comprises at least 3 amino acid residues, 3 to 20 amino acid residues, or 3 to 10 amino acid residues.

In one example, the first linker L1 has an amino acid sequence shown in any one of SEQ ID NOs: 10, 11, 15, 16, 18 to 20, 31, and 43, or has an amino acid sequence with at least 80%, 85%, 90%, 95%, or 99% sequence identity to any one of SEQ ID NOs: 10, 11, 15, 16, 18 to 20, 31, and 43.

The toxin molecule is any one of the following toxic polypeptides: (a) a plant-derived toxin; (b) a bacterial-derived toxin; (c) a fungal-derived toxin; (d) a human-derived protein toxin; (e), (f) a functional fragment derived from any one of (a) to (d) or a variant of the functional fragment. That is, the toxin molecule may be the entire toxin, or merely a functional fragment of the toxin capable of exerting the function of killing target cells.

The toxic polypeptide from a certain source may be an existing toxin or a variant thereof. For example, the plant-derived toxin may include an existing plant-derived toxin or a variant of these known plant-derived toxins.

Preferably, the toxin molecule is derived from: Diphtheria toxin, Pseudomonas exotoxin A (PE38), or Cholerae toxin. This illustrates the specific types of toxins from which the toxin molecule is derived. Specifically, the toxin molecule is derived from: Diphtheria toxin, a functional fragment of Diphtheria toxin, a variant of Diphtheria toxin, a variant of the functional fragment of Diphtheria toxin, Pseudomonas exotoxin A, a functional fragment of Pseudomonas exotoxin A, a variant of Pseudomonas exotoxin A, a variant of the functional fragment of Pseudomonas exotoxin A, Cholerae toxin, a functional fragment of Cholerae toxin, a variant of Cholerae toxin, or a variant of the functional fragment of Cholerae toxin.

The toxin comprises three domains: a binding domain (B domain), a translocation domain (T domain), and a catalytic domain (A domain). The binding domain binds to a receptor or antigen on the surface of the target cell. The translocation domain is transported into the cytoplasm through internalization and transmembrane translocation. The catalytic domain inhibits protein synthesis or induces apoptosis through intracellular catalysis, thereby achieving the killing effect on the target cell.

As used herein, a functional fragment of the toxin is, for example, only the catalytic domain A of the toxin, for another example, a combination of the translocation domain T and the catalytic domain A of the toxin.

The structure of the toxin molecule comprised in the effector moiety is any one of the following:
(1) a translocation domain DT and a catalytic domain DA of Diphtheria toxin, preferably connected in an orientation of DA-DT from the N-terminus to the C-terminus;
(2) the catalytic domain DA of Diphtheria toxin;
(3) a translocation domain PT and a catalytic domain PA of Pseudomonas exotoxin A, preferably connected in an orientation of PT-PA from the N-terminus to the C-terminus;
(4) the catalytic domain PA of Pseudomonas exotoxin A;
(5) a translocation domain CT and a catalytic domain CA of Cholerae toxin, preferably connected in an orientation of CT-CA from the N-terminus to the C-terminus;
(6) the catalytic domain CA of Cholerae toxin.

In one example, the toxin molecule has an amino acid sequence shown in any one of SEQ ID NOs: 1 to 9, or has an amino acid sequence with at least 80%, 85%, 90%, 95%, or 99% sequence identity to any one of SEQ ID NOs: 1 to 9.

In one example, the targeting vector moiety is an antibody, wherein the antibody is any one or more of a monoclonal antibody, a chimeric antibody, a reshaped antibody, a small molecule antibody, a multispecific antibody, and a multivalent antibody.

In one example, the antibody is any one or more of a small molecule antibody or a multivalent antibody.

Preferably, the small molecule antibody is any one or more of Fv, Fab, ScFv, ScFab, ScIgG, dsFv, and a single-domain antibody.

In one example, the light chain portion and the heavy chain portion of each of ScFab, ScFv, and ScIgG are connected via a second linker L2, for example:
the structural formula of ScFab is, for example, L-L2-VH-CH1 or VH-CH1-L2-L;
the structural formula of ScFv is, for example, VH-L2-VL;
the structural formula of ScIgG is, for example, L-L2-H.

The second linker comprises at least 25 amino acid residues, preferably 28 to 180, 30 to 120, 30 to 90, 30 to 85, or 30 to 82 amino acid residues.

In one example, the second linker L2 comprises any one or more of predominant amino acids of G, S, and T, and the number of the predominant amino acids accounts for at least more than 40%, further, more than 45%, and more preferably, more than 60% of the total number of amino acids in the second linker.

Further, the second linker L2 has an amino acid sequence shown in any one of SEQ ID NOs: 10, 12, 13, 14, 17, 28, 45, and 47, or has an amino acid sequence with at least 80%, 85%, 90%, 95%, or 99% sequence identity to any one of SEQ ID NOs: 10, 12, 13, 14, 17, 28, 45, and 47.

In one example, the structure of ScFab satisfies the following: the light chain portion and the heavy chain portion of ScFab are connected in an orientation from the N-terminus to the C-terminus, or the heavy chain portion and the light chain portion of ScFab are connected in an orientation from the N-terminus to the C-terminus.

In one example, the heavy chain portion and the light chain portion of ScFv are connected in an orientation from the N-terminus to the C-terminus.

Preferably, the multivalent antibody is a tandem antibody formed by at least two single-chain antibodies in tandem, such as two ScFvs in tandem or two ScFabs in tandem. There may be a linker or no linker between two unit structures in tandem.

Further preferably, the multivalent antibody is an antibody polymeric fusion protein formed by a small molecule antibody and a polymeric protein, wherein multiple small molecule antibodies are polymerized into a multimer via the polymeric protein; preferably, the antibody polymeric fusion protein is a dimer or a tetramer.

Further, examples of the antibody polymeric fusion protein include: a fusion of ScFab with an antibody fragment CH3, a fusion of ScFv with an antibody fragment CH3, a fusion of ScFab with Tamavidins2 protein, and a fusion of ScFv with Tamavidins2 protein.

Tamavidins is an antifungal protein, which is a novel streptavidin-like protein purified from the edible mushroom *Pleurotus cornucopiae* (Tamogitake) according to U.S. Patent No. 12/716,182. The gene derived from the purified protein is referred to as Tam1, the protein having the amino acid sequence encoded by Tam1 is referred to as Tamavidins1, the homolog of Tam1 is referred to as Tam2, and the protein having the amino acid sequence encoded by Tam2 is referred to as Tamavidins2. Tamavidins1 and Tamavidins2 exhibit 46.7% and 48.1% homology, respectively, with streptavidin in amino acid sequence, and 31.2% and 36.2% homology, respectively, with avidin in amino acid sequence.

Preferably, the fusion of ScFab with Tamavidins2 protein is connected in an orientation from the N-terminus to the C-terminus as Tamavidins2 protein to ScFab;
the fusion of ScFv with Tamavidins2 protein is connected in an orientation from the N-terminus to the C-terminus as Tamavidins2 protein to ScFv.

ScFab and the antibody fragment CH3 are connected in an orientation from the N-terminus to the C-terminus or from the C-terminus to the N-terminus.

In one example, the light chain portion constituting the Fab or ScFab has an amino acid sequence shown in SEQ ID NO: 32, or has an amino acid sequence with at least 80%, 85%, 90%, 95%, or 99% sequence identity to SEQ ID NO: 32.

In one example, the variable domain of the heavy chain portion constituting the Fab or ScFab has an amino acid sequence shown in SEQ ID NO: 33 or 55, or has an amino acid sequence with at least 80%, 85%, 90%, 95%, or 99% sequence identity to SEQ ID NO: 33 or 55.

In one example, the first constant domain CH1 of the heavy chain portion constituting the Fab or ScFab has an amino acid sequence shown in SEQ ID NO: 34 or 57, or has an amino acid sequence with at least 80%, 85%, 90%, 95%, or 99% sequence identity to SEQ ID NO: 34 or 57.

In one example, the variable domain of the light chain portion constituting the Fv, dsFv, or ScFv has an amino acid sequence shown in SEQ ID NO: 35 or 54, or has an amino acid sequence with at least 80%, 85%, 90%, 95%, or 99% sequence identity to SEQ ID NO: 35 or 54.

In one example, the variable domain of the heavy chain portion constituting the Fv, dsFv, or ScFv has an amino acid sequence shown in SEQ ID NO: 33 or 55, or has an amino acid sequence with at least 80%, 85%, 90%, 95%, or 99% sequence identity to SEQ ID NO: 33 or 55.

In one example, the light chain portion constituting the ScIgG has an amino acid sequence shown in SEQ ID NO: 32 or 56, or has an amino acid sequence with at least 80%, 85%, 90%, 95%, or 99% sequence identity to SEQ ID NO: 32 or 56.

In one example, the heavy chain portion constituting the ScIgG has an amino acid sequence shown in SEQ ID NO: 36 or 17, or has an amino acid sequence with at least 80%, 85%, 90%, 95%, or 99% sequence identity to SEQ ID NO: 36 or 17.

In one example, in the fusion protein provided by the present disclosure, the antibody fragment CH3 involved has the amino acid sequence shown in SEQ ID NO: 37, or has an amino acid sequence with at least 80%, 85%, 90%, 95%, or 99% sequence identity to SEQ ID NO: 37.

In one example, in the fusion protein provided by the present disclosure, the Tamavidins2 protein involved has the amino acid sequence shown in SEQ ID NO: 38, or has an amino acid sequence with at least 80%, 85%, 90%, 95%, or 99% sequence identity to SEQ ID NO: 38.

In one example, the nanobody involved has an amino acid sequence shown in SEQ ID NO: 52 or 53, or has an amino acid sequence with at least 80%, 85%, 90%, 95%, or 99% sequence identity to SEQ ID NO: 52 or 53.

Two single-chain antibodies, or a small molecule antibody and a polymeric protein, are connected via a third linker L3, for example:
the structural formula of two ScFabs in tandem is, for example:
   ScFab (L-L2-VH-CH1)-L3-ScFab (L-L2-VH-CH1);
the structural formula of two ScFvs in tandem is, for example:
   ScFv (VH-L2-VL)-L3-ScFv (VH-L2-VL);
the structural formula of the fusion of ScFab with the antibody fragment CH3 is, for example:
   CH3-L3-ScFab (L-L2-VH-CH1);
the structural formula of the fusion of ScFv with the antibody fragment CH3 is, for example:
   CH3-L3-ScFv (VH-L2-VL);
the structural formula of the fusion of Tamavidins2 protein with ScFab is, for example:
   Tamavidins2-L3-ScFab (L-L2-VH-CH1).

The third linker L3 is absent or comprises at least 3 amino acid residues; preferably, the third linker L3 comprises 5 to 20 amino acid residues.

In one example, the third linker L3 comprises two predominant amino acid residues, G and S, and the number of the predominant amino acid residues accounts for more than 40% of the total number of amino acid residues in the third linker; preferably, the proportion is more than 50%, 60%, 65%, 70%, or 75%.

In one example, the third linker L3 is the hinge region of IgG1 or a fragment having at least 80%, at least 85%, at least 90%, at least 95%, or at least 99% sequence identity to the hinge region.

For example, the third linker L3 has an amino acid sequence shown in any one of SEQ ID NOs: 10, 14, 18, 20 to 30, 44, and 48 to 51, or has an amino acid sequence with at least 80%, 85%, 90%, 95%, or 99% sequence identity to any one of SEQ ID NOs: 10, 14, 18, 20 to 30, 44, and 48 to 51.

In one example, the antibody is an antibody that specifically binds to any one of Her2 and CD22.

The human epidermal growth factor receptor (HER) family includes four structurally related members: HER1 (ErbB1, also referred to as EGFR), HER2 (ErbB2, also referred to as HER2/neu), HER3 (ErbB3), and HER4 (ErbB4). HER2 is a 185 kDa protein comprising an extracellular ligand-binding domain (ECD) and an intracellular tyrosine kinase domain. The N-terminal ECD can be further divided into four subdomains (I to IV). HER2 is considered a promising therapeutic target for breast cancer, as it has been found to be overexpressed in approximately one-quarter of breast cancer patients (Bange *et al.,* 2001, *Nature Medicine* 7: 548). *In vitro* studies have demonstrated that inhibition of HER2 can induce significant apoptosis in breast cancer cells (Faltus T et al., Neoplasia. 2004; 6(6): 786-95; Yang G et al., J Biol Chem. 2004; 279(6): 4339-45). In recent years, HER2 has emerged as an important biomarker and therapeutic target in approximately 30% of breast cancer patients.

CD22 is a lineage-restricted B-cell antigen expressed in 60 to 70% of B-cell lymphomas and leukemias. CD22 is absent from the cell surface during the early stages of B-cell development and is not expressed on stem cells (Tedder, T. F. et al., Annu Rev Immunol, 5: 481-504 (1997)).

Further, the antibody is derived from IgM, IgG, IgA, IgD, and IgE;
preferably, the antibody is derived from Trastuzumab, Rituximab, Bevacizumab, Adalimumab, Pembrolizumab, Ustekinumab, Nivolumab, Ocrelizumab, Palivizumab, Infliximab, Omalizumab, Envafolimab, Atlizumab, Atezolizumab, or Cetuximab, or variants thereof.

In one example, the fusion protein provided by the present disclosure has an EC50 value of less than or equal to 1 nM, or less than or equal to 0.8 nM, or less than or equal to 0.7 nM, or less than or equal to 0.6 nM, or less than or equal to 0.5 nM, or less than or equal to 0.4 nM, or less than or equal to 0.2 nM.

In one example, the IC50 value of the fusion protein is comparable to the IC50 value of commercially available DS-8201a or T-DM1 (for the IC50 value of DS-8201a, see Yusuke Ogitani et al., DS-8201a, a novel HER2-targeting ADC with a novel DNA topoisomerase I inhibitor, demonstrates a promising anti-tumor efficacy with differentiation from T-DM1, March 29, 2016; DOI: 10.1158/1078-0432.CCR-15-2822; for the IC50 value of T-DM1, see Junttila TT, Li G, Parsons K, Phillips GL, Sliwkowski MX, Trastuzumab-DM1 (T-DM1) retains all the mechanisms of action of trastuzumab and efficiently inhibits growth of lapatinib insensitive breast cancer. Breast cancer research and treatment. 2011; 128: 347-56).

The fusion protein provided by the present disclosure is suitable for cell-free *in vitro* synthesis; preferably, the cell-free *in vitro* synthesis involves: in a cell-free *in vitro* synthesis system, using a coding nucleic acid of the fusion protein as a DNA template, incubating under certain conditions for a certain period of time to obtain the fusion protein.

In one example, the cell-free *in vitro* synthesis system comprises a cell extract, wherein the cell extract is derived from a yeast strain; further, the yeast cell is *Saccharomyces cerevisiae*, *Kluyveromyces*, or a combination thereof; in another preferred example, the *Kluyveromyces* is *Kluyveromyces lactis*, *Kluyveromyces marxianus*, *Kluyveromyces dobzhanskii*, or a combination thereof.

In one example, the yeast strain is genetically engineered to be toxin-resistant, and a cell extract obtained from a toxin-resistant strain can overcome the toxicity issues associated with immunotoxins when used for *in vitro* protein synthesis.

As used herein, "toxin-resistant" refers to a certain degree of tolerance to the toxicity of immunotoxins.

Preferably, the strain from which the cell extract is derived herein exhibits reduced expression or activity of the DPH gene in the cell and/or a mutation in the amino acid sequence of eEF2; preferably, the expression level of the DPH gene is 10% or less, preferably 5% or less, and more preferably 2% or less; and/or the reduction in expression or activity of the DPH gene satisfies the following condition: the ratio of A1/A0 is 30% or less, preferably 10% or less, more preferably 5% or less, even more preferably 2% or less, and most preferably 0 to 2%, wherein A1 represents the expression or activity of the DPH gene in the genetically engineered cell, and A0 represents the expression or activity of the wild-type DPH gene.

Further preferably, the reduction in expression or activity of the DPH gene in the cell is achieved by a method selected from the group consisting of: gene editing, gene mutation, gene knockout, gene disruption, RNA interference technology, or a combination thereof.

Further preferably, the DPH gene is one or more selected from DPH1 to DPH7 genes, specifically DPH1, DPH2, DPH3, DPH4, DPH5, DPH6, and DPH7 genes.

Further preferably, the amino acid sequence of eEF2 has a mutation at position 699 and/or position 701.

Further preferably, the histidine (H) at position 699 in the amino acid sequence of eEF2 is mutated to methionine (M).

Further preferably, the glycine (G) at position 701 in the amino acid sequence of eEF2 is mutated to arginine (R).

Further preferably, the cell contains at least a knockout of the DPH gene.

Further preferably, the cell contains both a knockout of the DPH gene and a mutation in the amino acid sequence of eEF2.

Further preferably, the amino acid sequence of the protein encoded by the DPH gene is SEQ ID NOs: 59 to 65, or a polypeptide having 85% or more, 90% or more, 95% or more, 97% or more, 98% or more, or 99% or more homology to the amino acid sequence shown in SEQ ID NOs: 59 to 65 and having the same activity as the sequence shown in SEQ ID NOs: 59 to 65.

Further preferably, the amino acid sequence of eEF2 is SEQ ID NO: 66, or a polypeptide having 85% or more, 90% or more, 95% or more, 97% or more, 98% or more, or 99% or more homology to the amino acid sequence shown in SEQ ID NO: 66 and having the same activity as the sequence shown in SEQ ID NO: 66.

### Nucleic acid

The present disclosure also provides an isolated nucleic acid. A "nucleic acid molecule" includes DNA (*e.g*., genomic DNA or complementary DNA) and mRNA molecules, which may be single-stranded or double-stranded.

"Isolated" means that the nucleic acid molecule is not located or otherwise not provided within a cell.

### Vector

The present disclosure also provides a vector comprising the nucleic acid described above.

In one example, the vector is an expression vector.

### Host cell

The present disclosure also provides a host cell comprising the nucleic acid described above or the vector described above.

Preferably, the host cell is a yeast cell.

Further, the yeast cell is *Saccharomyces cerevisiae*, *Kluyveromyces*, or a combination thereof; in another preferred example, the *Kluyveromyces* is *Kluyveromyces lactis, Kluyveromyces marxianus*, *Kluyveromyces dobzhanskii*, or a combination thereof.

In one example, the strain from which the yeast cell is derived has been genetically engineered to be toxin-resistant as described above.

### In vitro cell-free protein synthesis system

The present disclosure also provides an *in vitro* cell-free protein synthesis system for synthesizing the fusion protein using an mRNA or DNA encoding the fusion protein according to any one of claims 1 to 17 as a template.

In one example, the *in vitro cell-free* protein synthesis system provided by the present disclosure comprises any one or more of the following:
(1) an mRNA or DNA template encoding the fusion protein described above;
(2) a cell extract, wherein the cell extract is a yeast cell extract; further, the cell extract is derived from a strain selected from the group consisting of *Saccharomyces cerevisiae*, *Pichia pastoris*, and *Kluyveromyces,* or a combination thereof; preferably, the yeast cell extract is derived from *Kluyveromyces,* more preferably *Kluyveromyces lactis*; more preferably, the yeast cell extract is derived from *Kluyveromyces,*
(3) any one or more of the following components:
   an amino acid mixture, dNTP, RNA polymerase, DNA polymerase, an energy supply system, polyethylene glycol, and an aqueous solvent.

In one example, in the *in vitro* cell-free protein synthesis system, the strain from which the cell extract is derived has been genetically engineered to be toxin-resistant as described above.

### In vitro cell-free synthesis method

The present disclosure also provides an *in vitro* cell-free synthesis method for the fusion protein described above, comprising:
in a reaction system comprising the *in vitro* cell-free protein synthesis system described above, performing an *in vitro* synthesis reaction using an mRNA or DNA encoding the fusion protein described above as a template to obtain the fusion protein; preferably, the DNA template and other components involved in the *in vitro* synthesis reaction have a volume ratio of 1:10 to 1:50, preferably 1:20 to 1:40, most preferably 1:25 to 1:35, and particularly preferably 1:30.

In one example, in the *in vitro* cell-free synthesis method provided by the present disclosure, the *in vitro* cell-free protein synthesis system comprises a yeast cell extract, an amino acid mixture, dNTP, RNA polymerase, DNA polymerase, an energy supply system, polyethylene glycol, and an aqueous solvent;
further:
the yeast cell extract is derived from a strain selected from the group consisting of *Saccharomyces cerevisiae*, *Pichia pastoris*, and *Kluyveromyces,* or a combination thereof; preferably, the yeast cell extract is derived from *Kluyveromyces,* more preferably *Kluyveromyces lactis*;
further, the strain is genetically engineered to be toxin-resistant.

In one example, in the *in vitro* cell-free synthesis method provided by the present disclosure, the yeast cell extract accounts for 50 to 80%, or 50% to 55%, or 50 to 60%, or 50 to 65% of the total volume of the *in vitro* cell-free protein synthesis system.

### Use in manufacturing fusion protein

The present disclosure also provides a use of the nucleic acid described above in the manufacture of the fusion protein described above.

The present disclosure also provides a use of the vector described above in the manufacture of the fusion protein described above.

The present disclosure also provides a use of the host cell described above in the manufacture of the fusion protein described above.

The present disclosure also provides a use of the *in vitro* cell-free protein synthesis system described above in the manufacture of the fusion protein described above.

### Use in disease treatment

The present disclosure also provides a use of the fusion protein described above in disease treatment, preferably for use in anti-tumor therapy, anti-transplant rejection, and treatment of an autoimmune disease.

In one example, the disease treatment is a treatment targeting HER2.

The fusion protein described above provided by the present disclosure exhibits an affinity EC50 value of 0.16 nM for target sites, such as HER2, and a cytotoxicity IC50 value for target cells comparable to that of existing products, and may be used in the disease treatment described above.

The fusion protein may be used preferably for the treatment of resistant cancer selected from lung cancer, urothelial cancer, colorectal cancer, prostate cancer, ovarian cancer, pancreatic cancer, breast cancer, bladder cancer, gastric cancer, gastrointestinal stromal tumor, cervical cancer, esophageal cancer, squamous cell carcinoma, peritoneal cancer, liver cancer, hepatocellular carcinoma, colon cancer, rectal cancer, colorectal cancer, endometrial cancer, uterine cancer, salivary gland cancer, renal cancer, vulvar cancer, thyroid cancer, penile cancer, leukemia, malignant lymphoma, plasmacytoma, myeloma, or sarcoma; more preferably for the treatment of resistant cancer selected from breast cancer, gastric cancer, colorectal cancer, or non-small cell lung cancer; and even more preferably for the treatment of resistant cancer selected from breast cancer or gastric cancer.

### Use in manufacturing medicament

The present disclosure also provides a use of the nucleic acid described above in the manufacture of a medicament for use in the disease treatment described above.

The present disclosure also provides a use of the vector described above in the manufacture of a medicament for use in the disease treatment described above.

The present disclosure also provides a use of the host cell described above in the manufacture of a medicament for use in the disease treatment described above.

The present disclosure also provides a use of the fusion protein described above in the manufacture of a medicament for use in the disease treatment described above.

The present disclosure is further described below with reference to specific examples.

### Example 1: Molecular design of fusion protein of the present disclosure

The fusion protein was designed by using active fragments of Diphtheria toxin and PE38 as the effector moiety A, and antibodies targeting Her2 and CD22 as the targeting vector moiety B, respectively.

The effector moiety A and the targeting vector moiety B were connected via a first linker L1, whose length and sequence composition were optimized.

For Diphtheria toxin, a fragment comprising the catalytic domain A and the translocation domain T was selected, with the binding domain capable of recognizing the natural target deleted. Specifically, the fragment corresponds to amino acids 1 to 389.

For PE toxin, which comprises three domains, including domain I at the N-terminus responsible for binding to the natural target, domain II in the middle responsible for transmembrane targeting (translocation domain PT), and domain III at the C-terminus being the catalytic domain (catalytic domain PA), a fragment comprising the translocation domain PTI and the catalytic domain PAI was selected, with domain I deleted. Specifically, the fragment corresponds to amino acids 251 to 613.

Trastuzumab is a humanized IgG1 monoclonal antibody targeting Her2, whose Fv fragment, Fab fragment, or full-length antibody was selected as the targeting vector moiety B. The fragment or full-length antibody of Trastuzumab was designed as a single-chain molecule, including single-chain Fv (ScFv), single-chain Fab (ScFab), and single-chain IgG (ScIgG). Specifically, the ScFv was formed by connecting VL and VH (VL-VH) or VH and VL (VH-VL), the ScFab was formed by connecting the light chain with the VH-CH1 fragment, and the ScIgG was formed by connecting the light chain with the heavy chain, wherein these connections were achieved via a second linker L2, whose length and amino acid sequence were optimized.

The CD22 antibody employed was derived from the antibody portion of the FDA-approved ADC drug Inotuzumab ozogamicin, which is a humanized IgG4 monoclonal antibody targeting CD22. The specific design of the single-chain molecule followed the design principles described above for the humanized IgG1 monoclonal antibody targeting Her2.

To enhance the affinity of the targeting moiety B for the target, the ScFvs or ScFabs in the immunotoxin were tandemly arranged to form a molecular construct of ScFv-ScFv or ScFab-ScFab, wherein the ScFvs in tandem or ScFabs in tandem were connected via a third linker L3. Additionally, CH3 and Tam2, which mediate dimerization and tetramerization, respectively, were fused with ScFv and ScFab to form molecular constructs including ScFv-CH3, CH3-ScFv, ScFab-CH3, CH3-ScFab, ScFv-Tam2, Tam2-ScFv, ScFab-Tam2, and Tam2-ScFab, wherein these fusions with CH3 and Tam2 were achieved via the third linker L3, whose length and amino acid sequence were optimized.

The specific molecular design information of the fusion proteins is shown in Table 1, and the sequences involved in each portion of the molecule are detailed in Table 6.

| Table 1 | | | | | |
|---|---|---|---|---|---|
| "/" in the table indicates no linker | | | | | |
| DT-HER2 antibody | | | | | |
| No. | Molecule ID | Molecular construct | L1 (Linker 1) sequence | L2 (Linker 2) sequence | L3 (Linker 3) sequence |
| 1 | DTH2-1 | DT-L1-ScFv (VH-L2-VL) | SEQ ID NO: 11 | SEQ ID NO: 28 | |
| 2 | DTH2-10 | DT-L1-ScFab (L-L2-VH-CH1)-L3-ScFab | SEQ ID NO: 10 | SEQ ID NO: 12 | SEQ ID NO: 25 |
| 3 | DTH2-104 | DT-L1-(VHH1-L3-CH3-L3-VHH2) | SEQ ID NO: 11 | | SEQ ID NO: 10 |
| 4 | DTH2-105 | DT-L1-(VHH1-L3-CH3-L3-VHH2) | SEQ ID NO: 10 | | SEQ ID NO: 10 |
| 5 | DTH2-106 | DT-L1-VHH1 | SEQ ID NO: 11 | | |
| 6 | DTH2-108 | DT-L1-VHH1-L3-CH3 | SEQ ID NO: 11 | | SEQ ID NO: 10 |
| 7 | DTH2-109 | DT-L1-VHH2 | SEQ ID NO: 11 | | |
| 8 | DTH2-11 | DT-L1-ScFab (L-L2-VH-CH1)-L3-ScFab | SEQ ID NO: 10 | SEQ ID NO: 12 | SEQ ID NO: 26 |
| 9 | DTH2-110 | DT-L1-VHH2 | SEQ ID NO: 10 | | |
| 10 | DTH2-111 | DT-L1-VHH2-L3-CH3 | SEQ ID NO: 11 | | SEQ ID NO: 10 |
| 11 | DTH2-12 | DT-L1-ScFab (L-L2-VH-CH1)-L3-ScFab | SEQ ID NO: 11 | SEQ ID NO: 12 | SEQ ID NO: 24 |
| 12 | DTH2-13 | DT-L1-ScFv (VH-L2-VL)-L3-ScFv | SEQ ID NO: 11 | SEQ ID NO: 28 | SEQ ID NO: 29 |
| 13 | DTH2-14 | DT-L1-ScFv (VH-L2-VL)-L3-ScFv | SEQ ID NO: 10 | SEQ ID NO: 28 | SEQ ID NO: 27 |
| 14 | DTH2-15 | DT-L1-ScFv (VL-L2-VH)-L3-ScFv | SEQ ID NO: 11 | SEQ ID NO: 14 | SEQ ID NO: 30 |
| 15 | DTH2-16 | DT-L1-ScFv (VL-L2-VH)-L3-ScFv | SEQ ID NO: 10 | SEQ ID NO: 14 | SEQ ID NO: 27 |
| 16 | DTH2-17 | DT-L1-ScFab (L-L2-VH-CH1)-L3-ScFab (L-L2-VH-CH1) | SEQ ID NO: 11 | SEQ ID NO: 12 | SEQ ID NO: 30 |
| 17 | DTH2-18 | DT-L1-ScFab (L-L2-VH-CH1)-L3-ScFab | SEQ ID NO: 10 | SEQ ID NO: 12 | SEQ ID NO: 27 |
| 18 | DTH2-2 | DT-L1-ScFv (VH-L2-VL) | SEQ ID NO: 10 | SEQ ID NO: 28 | |
| 19 | DTH2-23 | DT-L1-CH3-L3-ScFab (L-L2-VH-CH1) | SEQ ID NO: 11 | SEQ ID NO: 12 | SEQ ID NO: 22 |
| 20 | DTH2-25 | DT-L1-ScFab (L-L2-VH-CH1)-L3-CH3 | SEQ ID NO: 11 | SEQ ID NO: 12 | SEQ ID NO: 22 |
| 21 | DTH2-26 | DT-L1-ScFab (L-L2-VH-CH1)-L3-CH3 | SEQ ID NO: 11 | SEQ ID NO: 12 | SEQ ID NO: 51 |
| 22 | DTH2-28 | DT-L1-Tam2-L3-ScFab (L-L2-VH-CH1) | SEQ ID NO: 11 | SEQ ID NO: 12 | SEQ ID NO: 22 |
| 23 | DTH2-3 | DT-L1-ScFab (L-L2-VH-CH1) | SEQ ID NO: 11 | SEQ ID NO: 12 | |
| 24 | DTH2-32 | DT-L1-CH3-L3-ScFv (VH-L2-VL) | SEQ ID NO: 11 | SEQ ID NO: 28 | SEQ ID NO: 22 |
| 25 | DTH2-34 | DT-L1-ScFv (VH-L2-VL)-L3-CH3 | SEQ ID NO: 11 | SEQ ID NO: 28 | SEQ ID NO: 22 |
| 26 | DTH2-36 | DT-L1-Tam2-L3-ScFv (VH-L2-VL) | SEQ ID NO: 11 | SEQ ID NO: 28 | SEQ ID NO: 22 |
| 27 | DTH2-38 | DT-L1-ScFv (VH-L2-VL)-L3-Tam2 | SEQ ID NO: 11 | SEQ ID NO: 28 | SEQ ID NO: 22 |
| 28 | DTH2-4 | DT-L1-ScFab (L-L2-VH-CH1) | SEQ ID NO: 10 | SEQ ID NO: 12 | |
| 29 | DTH2-42 | DT-L1-CH3-L3-ScFv (VL-L2-VH) | SEQ ID NO: 11 | SEQ ID NO: 28 | SEQ ID NO: 22 |
| 30 | DTH2-44 | DT-L1-ScFv (VL-L2-VH)-L3-CH3 | SEQ ID NO: 11 | SEQ ID NO: 28 | SEQ ID NO: 22 |
| 31 | DTH2-46 | DT-L1-ScFv (VL-L2-VH)-L3-Tam2 | SEQ ID NO: 11 | SEQ ID NO: 28 | SEQ ID NO: 22 |
| 32 | DTH2-48 | DT-L1-ScFab (L-L2-VH-CH1)-L3-CH3 | SEQ ID NO: 11 | SEQ ID NO: 12 | SEQ ID NO: 20 |
| 33 | DTH2-49 | DT-L1-ScFab (L-L2-VH-CH1)-L3-CH3 | SEQ ID NO: 11 | SEQ ID NO: 12 | SEQ ID NO: 21 |
| 34 | DTH2-5 | DT-L1-ScIgG (L-L2-H) | SEQ ID NO: 11 | SEQ ID NO: 12 | |
| 35 | DTH2-50 | DT-L1-ScFab (L-L2-VH-CH1)-L3-CH3 | SEQ ID NO: 11 | SEQ ID NO: 12 | / |
| 36 | DTH2-51 | DT-L1-ScFab (L-L2-VH-CH1)-L3-CH3 | SEQ ID NO: 11 | SEQ ID NO: 12 | / |
| 37 | DTH2-6 | DT-L1-ScIgG (L-L2-H) | SEQ ID NO: 10 | SEQ ID NO: 12 | |
| 38 | DTH2-62 | DT-L1-ScFab (L-L2-VH-CH1)-L3-ScFab | SEQ ID NO: 11 | SEQ ID NO: 13 | SEQ ID NO: 28 |
| 39 | DTH2-63 | DT-L1-ScFab (L-L2-VH-CH1)-L3-ScFab | SEQ ID NO: 10 | SEQ ID NO: 13 | SEQ ID NO: 28 |
| 40 | DTH2-64 | DT-L1-ScFab (L-L2-VH-CH1)-L3-CH3 | SEQ ID NO: 11 | SEQ ID NO: 12 | SEQ ID NO: 22 |
| 41 | DTH2-65 | DT-L1-ScFab (L-L2-VH-CH1)-L3-CH3 | SEQ ID NO: 11 | SEQ ID NO: 12 | SEQ ID NO: 22 |
| 42 | DTH2-66 | DT-L1-ScFab (L-L2-VH-CH1)-L3-CH3 | SEQ ID NO: 11 | SEQ ID NO: 12 | SEQ ID NO: 22 |
| 43 | DTH2-67 | DT-L1-ScFab (L-L2-VH-CH1)-L3-CH3 | SEQ ID NO: 11 | SEQ ID NO: 12 | SEQ ID NO: 22 |
| 44 | DTH2-68 | DT-L1-ScFab (L-L2-VH-CH1)-L3-CH3 | SEQ ID NO: 11 | SEQ ID NO: 12 | SEQ ID NO: 22 |
| 45 | DTH2-69 | DT-L1-ScFab (L-L2-VH-CH1)-L3-CH3 | SEQ ID NO: 11 | SEQ ID NO: 12 | SEQ ID NO: 14 |
| 46 | DTH2-7 | DT-L1-ScFv (VL-L2-VH) | SEQ ID NO: 11 | SEQ ID NO: 14 | |
| 47 | DTH2-70 | DT-L1-ScFab (L-L2-VH-CH1)-L3-CH3 | SEQ ID NO: 11 | SEQ ID NO: 12 | SEQ ID NO: 28 |
| 48 | DTH2-71 | DT-L1-ScFab (L-L2-VH-CH1)-L3-CH3 | SEQ ID NO: 11 | SEQ ID NO: 12 | SEQ ID NO: 50 |
| 49 | DTH2-72 | DT-L1-ScFab (L-L2-VH-CH1)-L3-CH3 | SEQ ID NO: 11 | SEQ ID NO: 12 | SEQ ID NO: 18 |
| 50 | DTH2-73 | DT-L1-ScFab (L-L2-VH-CH1)-L3-CH3 | SEQ ID NO: 11 | SEQ ID NO: 12 | SEQ ID NO: 48 |
| 51 | DTH2-74 | DT-L1-ScFab (L-L2-VH-CH1)-L3-CH3 | SEQ ID NO: 11 | SEQ ID NO: 12 | SEQ ID NO: 49 |
| 52 | DTH2-75 | DT-L1-ScFab (L-L2-VH-CH1)-L3-CH3 | SEQ ID NO: 11 | SEQ ID NO: 12 | SEQ ID NO: 44 |
| 53 | DTH2-76 | DT-L1-ScFab (L-L2-VH-CH1) | SEQ ID NO: 10 | SEQ ID NO: 12 | |
| 54 | DTH2-77 | DT-L1-ScFab (L-L2-VH-CH1) | SEQ ID NO: 20 | SEQ ID NO: 12 | |
| 55 | DTH2-78 | DT-L1-ScFab (L-L2-VH-CH1) | SEQ ID NO: 15 | SEQ ID NO: 12 | |
| 56 | DTH2-79 | DT-L1-ScFab (L-L2-VH-CH1) | SEQ ID NO: 31 | SEQ ID NO: 12 | |
| 57 | DTH2-8 | DT-L1-ScFv (VL-L2-VH) | SEQ ID NO: 10 | SEQ ID NO: 14 | |
| 58 | DTH2-80 | DT-L1-ScFab (L-L2-VH-CH1) | SEQ ID NO: 18 | SEQ ID NO: 12 | |
| 59 | DTH2-81 | DT-L1-ScFab (L-L2-VH-CH1) | SEQ ID NO: 19 | SEQ ID NO: 12 | |
| 60 | DTH2-82 | DT-L1-ScFab (L-L2-VH-CH1)-L3-CH3 | SEQ ID NO: 10 | SEQ ID NO: 12 | SEQ ID NO: 22 |
| 61 | DTH2-83 | DT-L1-ScFab (L-L2-VH-CH1)-L3-CH3 | SEQ ID NO: 10 | SEQ ID NO: 12 | SEQ ID NO: 22 |
| 62 | DTH2-84 | DT-L1-ScFab (L-L2-VH-CH1)-L3-CH3 | SEQ ID NO: 20 | SEQ ID NO: 12 | SEQ ID NO: 22 |
| 63 | DTH2-85 | DT-L1-ScFab (L-L2-VH-CH1)-L3-CH3 | SEQ ID NO: 15 | SEQ ID NO: 12 | SEQ ID NO: 22 |
| 64 | DTH2-86 | DT-L1-ScFab (L-L2-VH-CH1)-L3-CH3 | SEQ ID NO: 16 | SEQ ID NO: 12 | SEQ ID NO: 22 |
| 65 | DTH2-87 | DT-L1-ScFab (L-L2-VH-CH1)-L3-CH3 | SEQ ID NO: 18 | SEQ ID NO: 12 | SEQ ID NO: 22 |
| 66 | DTH2-88 | DT-L1-ScFab (L-L2-VH-CH1)-L3-CH3 | SEQ ID NO: 19 | SEQ ID NO: 12 | SEQ ID NO: 22 |
| 67 | DTH2-9 | DT-L1-ScFab (L-L2-VH-CH1)-L3-ScFab | SEQ ID NO: 11 | SEQ ID NO: 12 | SEQ ID NO: 23 |

| DT-CD22 antibody | | | | | |
|---|---|---|---|---|---|
| No. | Molecule ID | Molecular construct | L1 (Linker 1) sequence | L2 (Linker 2) sequence | L3 (Linker 3) sequence |
| 68 | DT22-12 | DT-L1-ScFv (VL-L2-VH)-SS | SEQ ID NO: 10 | SEQ ID NO: 28 | |
| 69 | DT22-14 | DT-L1-ScFv (VL-L2-VH)-SS | SEQ ID NO: 10 | SEQ ID NO: 47 | |
| 70 | DT22-2 | DT-L1-ScFv (VL-L2-VH) | SEQ ID NO: 10 | SEQ ID NO: 47 | |
| 71 | DT22-22 | DT-L1-ScFv (VH-L2-VL)-SS | SEQ ID NO: 10 | SEQ ID NO: 47 | |
| 72 | DT22-24 | DT-L1-ScFv (VH-L2-VL)-SS | SEQ ID NO: 10 | SEQ ID NO: 28 | |
| 73 | DT22-28 | DT-L1-ScFab (L-L2-VH-CH1) | SEQ ID NO: 10 | SEQ ID NO: 46 | |
| 74 | DT22-34 | DT-L1-ScIgG (L-L2-H) | SEQ ID NO: 10 | SEQ ID NO: 45 | |
| 75 | DT22-4 | DT-L1-ScFv (VL-L2-VH) | SEQ ID NO: 10 | SEQ ID NO: 28 | |

| PE38-Her2 antibody | | | | | |
|---|---|---|---|---|---|
| No. | Molecule ID | Molecular construct | L1 (Linker 1) sequence | L2 (Linker 2) sequence | L3 (Linker 3) sequence |
| 76 | PEH2-1 | ScFv (VL-L2-VH)-L1-PE38 | SEQ ID NO: 43 | SEQ ID NO: 14 | |
| 77 | PEH2-2 | ScFv (VL-L2-VH)-L1-PE38 | SEQ ID NO: 20 | SEQ ID NO: 14 | |
| 78 | PEH2-3 | ScFv (VH-L2-VL)-L1-PE38 | SEQ ID NO: 43 | SEQ ID NO: 14 | |
| 79 | PEH2-4 | ScFv (VH-L2-VL)-L1-PE38 | SEQ ID NO: 20 | SEQ ID NO: 14 | |

In Table 1, for tandem antibodies, the structural description of one of them is omitted when their structures are identical. For example, in "DT-L1-ScFab (L-L2-VH-CH1)-L3-ScFab", the two ScFabs have identical structures, so the structural description of one of them is omitted. The same applies below.

### Example 2: Preparation of fusion protein

The following example uses fusion protein DTH2-13 as an illustration, and the experimental methods for other constructed fusion proteins are similar.

### 2.1 Construction of expression plasmid for fusion protein

First, an expression plasmid for 8His-DTH2-eGFP-8His was constructed. On this basis, an antibody or antibody fragment was inserted at the C-terminus of the DT fragment (effector moiety A) by homologous recombination.

The specific method was as follows:
First, the DNA sequence of ScFab was amplified from a plasmid containing the DNA sequence of Trastuzumab ScIgG using primers 1 and 2:
Primer 1:
   CTGGTGGTGGTGGTTCTGACATCCAAATGACCCAATCTCCATCTTC;
Primer 2:
   GTGGTGGTGAGAACCACCCTTGTCACAAGACTTTGGTTCAACCTTC.

Then, the expression plasmid for SUMO-DT-8His-eGFP-8His was subjected to PCR amplification using primers 3 and 4:
Primer 3:
   GAACCAAAGTCTTGTGACAAGGGTGGTTCTCACCACCACCAC;
Primer 4:
   GGAGATTGGGTCATTTGGATGTCAGAACCACCACCACCAGAACC.

The two amplified DNA fragments were mixed at a volume ratio of 1:1 with a total volume of 10 µL, followed by addition of 1 µL of DpnI restriction enzyme. The mixture was incubated at 37°C for 4 to 5 hours and then transformed into *E. coli* Dh5a competent cells. The transformed cells were plated onto solid LB medium containing ampicillin and incubated overnight at 37°C.

The next day, single colonies were picked from the solid medium and inoculated into 200 µL of LB medium, followed by incubation at 37°C with shaking for approximately 6 hours. The bacterial suspension was then subjected to sequencing.

The correct clones were inoculated into 5 mL of LB medium and incubated overnight at 37°C with shaking. The next day, the plasmid was extracted using a plasmid extraction kit to complete the construction of the expression plasmid.

### 2.2 In vitro synthesis of fusion protein for expression

The method was described using a 30 mL reaction system as an example.

First, an AMPi amplification system (commercially available from Kangma-Healthcode, Cat. No. PROTN_AMPiN10V03500) for the expression plasmid was prepared. 100 µL of AMPi reaction mixture was added to 900 µL of ultrapure water, followed by addition of approximately 0.5 µL of AMPiase and the expression plasmid of DTH2 as a template, resulting in a final concentration of 2 ng/µL. The mixture was incubated overnight at 37°C.

The AMPi amplification results were analyzed by DNA gel electrophoresis. After the expression plasmid was amplified, 1 mL of the AMPi amplification system was added to a 30 mL D2P *in vitro* protein synthesis system (IVTT) at a volume ratio of 1:30, followed by incubation at 30°C with shaking for 3 to 4 hours.

The *in vitro cell-free* protein synthesis system (IVTT) used comprised: 22 mM HEPES (pH 7.4), 30 to 150 mM potassium acetate, 1.0 to 5.0 mM magnesium acetate, 1.5 to 4 mM nucleoside triphosphate mixture (ATP, GTP, CTP, and UTP), 0.08 to 0.24 mM amino acid mixture (glycine, alanine, valine, leucine, isoleucine, phenylalanine, proline, tryptophan, serine, tyrosine, cysteine, methionine, asparagine, glutamine, threonine, aspartic acid, glutamic acid, lysine, arginine, and histidine), 25 mM phosphocreatine, 1.7 mM dithiothreitol, 0.27 mg/mL creatine phosphokinase, 0.027 to 0.054 mg/mL T7 RNA polymerase, 1% to 4% polyethylene glycol, 0.5% to 2% sucrose, and finally 50% to 80% (v/v) yeast cell extract.

The yeast cell extract used was derived from *Kluyveromyces lactis* with a dph2 knockout strain (dph2Δ mutant strain).

### 2.3 Purification of fusion protein

Ni magnetic beads were used at 0.2% of the IVTT volume and washed 2 to 3 times with ultrapure water for later use.

After 3 hours of *in vitro* translation and incubation, the beads were added to the reaction system and incubated for another 1 hour. Then, the beads were separated from the reaction system using a magnetic rack, followed by sequential washing with 1 mL of washing buffer containing 5 mM imidazole, 20 mM imidazole, and 60 mM imidazole for 2 times, 1 time, and 1 time, respectively. Finally, the protein was eluted with 2 to 5 mL of elution buffer containing 250 mM imidazole.

The washing and elution fractions were analyzed by SDS-PAGE for purity, and the expression level of the immunotoxin was calculated based on the fluorescence intensity of the fusion-expressed eGFP.

The expression results demonstrated that, for the various fusion proteins prepared by *in vitro* cell-free protein synthesis in the present disclosure, excluding abnormal results, rational design of linkers enabled the lowest expression level of the purified fusion protein to exceed 10 mg/L after 3 hours, with some fusion proteins having an expression level of approximately 2000 mg/L and 5000 mg/L after 3 hours. Compared to current cell expression systems, the systems described herein offer a shorter production cycle and lower cost. For example, the expression level of known CHO cell systems is limited to 2 to 5 mg/L. For another example, *Pichia pastoris* systems for secretory expression require induction for more than 42 hours to achieve an expression level of approximately 30 mg/L, along with the need for various culture conditions.

Expression and purification were performed for some of the molecules in Table 1, and the yield results are shown in Table 2.

| Table 2 | | | | | | | | |
|---|---|---|---|---|---|---|---|---|
| In Table 2, "0" indicates no linker or no expression/purification | | | | | | | | |
| Molecule ID | Molecular construct | L1 sequence | L1 lengt h | L2 sequence | L2 lengt h | L3 sequence | L3 lengt h | Expressio n level (mg/L) |
| DTH2-104 | DT-L1-(VHH1-L3-CH3-L3-VHH2) | EASGGPE | 7 | | | (GGGGS)2 | 10 | 725.187 |
| DTH2-105 | DT-L1-(VHH1-L3-CH3-L3-VHH2) | (GGGGS) 2 | 10 | | | (GGGGS)2 | 10 | 827.192 |
| DTH2-32 | DT-L1-CH3-L3-ScFv (VH-L2-VL) | EASGGPE | 7 | (GGGGS)4 | 20 | GGSGGGGS | 8 | 288.844 |
| DTH2-42 | DT-L1-CH3-L3-ScFv (VL-L2-VH) | EASGGPE | 7 | (GGGGS)4 | 20 | GGSGGGGS | 8 | 182.330 |
| DT22-28 | DT-L1-ScFab (L-L2-VH-CH1) | (GGGGS) 2 | 10 | GGSSGSGSGST GTSSSGTGTSA GTTGTSASTSG SGSGGGGGSGG GGSAGGTATAG ASSGS | 60 | | | 407 |
| DTH2-4 | DT-L1-ScFab (L-L2-VH-CH1) | (GGGGS) 2 | 10 | GGSSGSGSGST GTSSSGTGTSA GTTGTSASTSG SGSGGGGGSGG GGSAGGTATAG ASSGS | 60 | | | 1968.685 |
| DTH2-76 | DT-L1-ScFab (L-L2-VH-CH1) | GGSGGG GS | 8 | GGSSGSGSGST GTSSSGTGTSA GTTGTSASTSG SGSGGGGGSGG GGSAGGTATAG ASSGS | 60 | | | 43,932 |
| DTH2-77 | DT-L1-ScFab (L-L2-VH-CH1) | GGGGS | 5 | GGSSGSGSGST GTSSSGTGTSA GTTGTSASTSG SGSGGGGGSGG GGSAGGTATAG ASSGS | 60 | | | 21.098 |
| DTH2-78 | DT-L1-ScFab (L-L2-VH-CH1) | GS | 2 | GGSSGSGSGST GTSSSGTGTSA GTTGTSASTSG SGSGGGGGSGG GGSAGGTATAG ASSGS | 60 | | | 41.943 |
| DTH2-79 | DT-L1-ScFab (L-L2-VH-CH1) | KESGSVS SEQLA | 12 | GGSSGSGSGST GTSSSGTGTSA GTTGTSASTSG SGSGGGGGSGG GGSAGGTATAG ASSGS | 60 | | | 0 |
| DTH2-80 | DT-L1-ScFab (L-L2-VH-CH1) | EGKSSGS GSES | 11 | GGSSGSGSGST GTSSSGTGTSA GTTGTSASTSG SGSGGGGGSGG GGSAGGTATAG ASSGS | 60 | | | 39.238 |
| DTH2-81 | DT-L1-ScFab (L-L2-VH-CH1) | GSAPAPA GS | 9 | GGSSGSGSGST GTSSSGTGTSA GTTGTSASTSG SGSGGGGGSGG GGSAGGTATAG ASSGS | 60 | | | 37.103 |
| DTH2-25 | DT-L1-ScFab (L-L2-VH-CH1)-L3-CH3 | EASGGPE | 7 | GGSSGSGSGST GTSSSGTGTSA GTTGTSASTSG SGSGGGGSGG GGSAGGTATAG ASSGS | 60 | GGSGGGGS | 8 | 516.288 |
| DTH2-26 | DT-L1-ScFab (L-L2-VH-CH1)-L3-CH3 | EASGGPE | 7 | GGSSGSGSGST GTSSSGTGTSA GTTGTSASTSG SGSGGGGGSGG GGSAGGTATAG ASSGS | 60 | CPPCPAPELL | 10 | 0 |
| DTH2-48 | DT-L1-ScFab (L-L2-VH-CH1)-L3-CH3 | EASGGPE | 7 | GGSSGSGSGST GTSSSGTGTSA GTTGTSASTSG SGSGGGGGSGG GGSAGGTATAG ASSGS | 60 | GGGGS | 5 | 0 |
| DTH2-49 | DT-L1-ScFab (L-L2-VH-CH1)-L3-CH3 | EASGGPE | 7 | GGSSGSGSGST GTSSSGTGTSA GTTGTSASTSG SGSGGGGGSGG GGSAGGTATAG ASSGS | 60 | GGS | 3 | 0 |
| DTH2-50 | DT-L1-ScFab (L-L2-VH-CH1)-L3-CH3 | EASGGPE | 7 | GGSSGSGSGST GTSSSGTGTSA GTTGTSASTSG SGSGGGGGSGG GGSAGGTATAG ASSGS | 60 | No linker | 0 | 0 |
| DTH2-51 | DT-L1-ScFab (L-L2-VH-CH1)-L3-CH3 | EASGGPE | 7 | GGSSGSGSGST GTSSSGTGTSA GTTGTSASTSG SGSGGGGGSGG GGSAGGTATAG ASSGS | 60 | No linker, with the first amino acid deleted at the N-terminus of CH3 | 0 | 0 |
| DTH2-64 | DT-L1-ScFab (L-L2-VH-CH1)-L3-CH3 | EASGGPE | 7 | GGSSGSGSGST GTSSSGTGTSA GTTGTSASTSG SGSGGGGGSGG GGSAGGTATAG ASSGS | 60 | GGSGGGGS, with SKAK added at the N-terminus of CH3 | 8 | 18.678 |
| DTH2-65 | DT-L1-ScFab (L-L2-VH-CH1)-L3-CH3 | EASGGPE | 7 | GGSSGSGSGST GTSSSGTGTSA GTTGTSASTSG SGSGGGGGSGG GGSAGGTATAG ASSGS | 60 | GGSGGGGS, with GPQ deleted at the N-terminus of CH3 | 8 | 26.548 |
| DTH2-66 | DT-L1-ScFab (L-L2-VH-CH1)-L3-CH3 | EASGGPE | 7 | GGSSGSGSGST GTSSSGTGTSA GTTGTSASTSG SGSGGGGGSGG GGSAGGTATAG ASSGS | 60 | GGSGGGGS, with KTHT deleted at the C-terminus of CH1 | 8 | 46.527 |
| DTH2-67 | DT-L1-ScFab (L-L2-VH-CH1)-L3-CH3 | EASGGPE | 7 | GGSSGSGSGST GTSSSGTGTSA GTTGTSASTSG SGSGGGGGSGG GGSAGGTATAG ASSGS | 60 | GGSGGGGS, with HT deleted at the C-terminus of CH1 | 8 | 36.172 |
| DTH2-68 | DT-L1-ScFab (L-L2-VH-CH1)-L3-CH3 | EASGGPE | 7 | GGSSGSGSGST GTSSSGTGTSA GTTGTSASTSG SGSGGGGGSGG GGSAGGTATAG ASSGS | 60 | GGSGGGGS, with G deleted at the N-terminus of CH3 | 8 | 36.225 |
| DTH2-69 | DT-L1-ScFab (L-L2-VH-CH1)-L3-CH3 | EASGGPE | 7 | GGSSGSGSGST GTSSSGTGTSA GTTGTSASTSG SGSGGGGGSGG GGSAGGTATAG ASSGS | 60 | (GGGGS)3 | 15 | 37.178 |
| DTH2-70 | DT-L1-ScFab (L-L2-VH-CH1)-L3-CH3 | EASGGPE | 7 | GGSSGSGSGST GTSSSGTGTSA GTTGTSASTSG SGSGGGGGSGG GGSAGGTATAG ASSGS | 60 | (GGGGS)4 | 20 | 40.140 |
| DTH2-71 | DT-L1-ScFab (L-L2-VH-CH1)-L3-CH3 | EASGGPE | 7 | GGSSGSGSGST GTSSSGTGTSA GTTGTSASTSG SGSGGGGGSGG GGSAGGTATAG ASSGS | 60 | KESGSVSSEQL A | 12 | 46.077 |
| DTH2-72 | DT-L1-ScFab (L-L2-VH-CH1)-L3-CH3 | EASGGPE | 7 | GGSSGSGSGST GTSSSGTGTSA GTTGTSASTSG SGSGGGGGSGG GGSAGGTATAG ASSGS | 60 | EGKSSGSGSE S | 11 | 12.735 |
| DTH2-73 | DT-L1-ScFab (L-L2-VH-CH1)-L3-CH3 | EASGGPE | 7 | GGSSGSGSGST GTSSSGTGTSA GTTGTSASTSG SGSGGGGGSGG GGSAGGTATAG ASSGS | 60 | GSAGSAAGSG EF | 12 | 0 |
| DTH2-74 | DT-L1-ScFab (L-L2-VH-CH1)-L3-CH3 | EASGGPE | 7 | GGSSGSGSGST GTSSSGTGTSA GTTGTSASTSG SGSGGGGGSGG GGSAGGTATAG ASSGS | 60 | GSAPAPAGGG GS | 12 | 60.139 |
| DTH2-75 | DT-L1-ScFab (L-L2-VH-CH1)-L3-CH3 | EASGGPE | 7 | GGSSGSGSGST GTSSSGTGTSA GTTGTSASTSG SGSGGGGGSGG GGSAGGTATAG ASSGS | 60 | GSAEAAAKEA AAKAGGGGS | 19 | 29.479 |
| DTH2-82 | DT-L1-ScFab (L-L2-VH-CH1)-L3-CH3 | (GGGGS) 2 | 10 | GGSSGSGSGST GTSSSGTGTSA GTTGTSASTSG SGSGGGGGSGG GGSAGGTATAG ASSGS | 60 | GGSGGGGS | 8 | 0 |
| DTH2-83 | DT-L1-ScFab (L-L2-VH-CH1)-L3-CH3 | GGSGGG GS | 8 | GGSSGSGSGST GTSSSGTGTSA GTTGTSASTSG SGSGGGGGSGG GGSAGGTATAG ASSGS | 60 | GGSGGGGS | 8 | 29.654 |
| DTH2-84 | DT-L1-ScFab (L-L2-VH-CH1)-L3-CH3 | GGGGS | 5 | GGSSGSGSGST GTSSSGTGTSA GTTGTSASTSG SGSGGGGGSGG GGSAGGTATAG ASSGS | 60 | GGSGGGGS | 8 | 109.868 |
| DTH2-85 | DT-L1-ScFab (L-L2-VH-CH1)-L3-CH3 | GS | 2 | GGSSGSGSGST GTSSSGTGTSA GTTGTSASTSG SGSGGGGGSGG GGSAGGTATAG ASSGS | 60 | GGSGGGGS | 8 | 38.133 |
| DTH2-86 | DT-L1-ScFab (L-L2-VH-CH1)-L3-CH3 | KESGSVS SEQLA | 12 | GGSSGSGSGST GTSSSGTGTSA GTTGTSASTSG SGSGGGGGSGG GGSAGGTATAG ASSGS | 60 | GGSGGGGS | 8 | 13.361 |
| DTH2-87 | DT-L1-ScFab (L-L2-VH-CH1)-L3-CH3 | EGKSSGS GSES | 11 | GGSSGSGSGST GTSSSGTGTSA GTTGTSASTSG SGSGGGGGSGG GGSAGGTATAG ASSGS | 60 | GGSGGGGS | 8 | 18.678 |
| DTH2-88 | DT-L1-ScFab (L-L2-VH-CH1)-L3-CH3 | GSAPAPA GS | 9 | GGSSGSGSGST GTSSSGTGTSA GTTGTSASTSG SGSGGGGGSGG GGSAGGTATAG ASSGS | 60 | GGSGGGGS | 8 | 16.589 |
| DTH2-10 | DT-L1-ScFab (L-L2-VH-CH1)-L3-ScFab | (GGGGS) 2 | 10 | GGSSGSGSGST GTSSSGTGTSA GTTGTSASTSG SGSGGGGGSGG GGSAGGTATAG ASSGS | 60 | GGSHHQPFEA SGGPE | 15 | 0 |
| DTH2-11 | DT-L1-ScFab CH1)-L3-ScFab | (GGGGS) 2 | 10 | GGSSGSGSGST GTSSSGTGTSA GTTGTSASTSG SGSGGGGGSGG GGSAGGTATAG ASSGS | 60 | GGSHHEASGG PE | 12 | 0 |
| DTH2-12 | DT-L1-ScFab CH1)-L3-ScFab | EASGGPE | 7 | GGSSGSGSGST GTSSSGTGTSA GTTGTSASTSG SGSGGGGGSGG GGSAGGTATAG ASSGS | 60 | GGSHHTQPFG GGGSGGGGS | 19 | 0 |
| DTH2-62 | DT-L1-ScFab (L-L2-VH-CH1)-L3-ScFab | EASGGPE | 7 | GAGAGSGAGAG SGAGAGSGAGA | 34 | (GGGGS)4 | 20 | 0 |
| | | | | GSGAGAGSGAG AGS | | | | |
| DTH2-63 | DT-L1-ScFab (L-L2-VH-CH1)-L3-ScFab | (GGGGS) 2 | 10 | GAGAGSGAGAG SGAGAGSGAGA GSGAGAGSGAG AGS | 34 | (GGGGS)4 | 20 | 0 |
| DTH2-9 | DT-L1-ScFab (L-L2-VH-CH1)-L3-ScFab | EASGGPE | 7 | GGSSGSGSGST GTSSSGTGTSA GTTGTSASTSG SGSGGGGGSGG GGSAGGTATAG ASSGS | 60 | GGSHHGHKT QPFGGGGSGG GGS | 22 | 0 |
| DTH2-18 | DT-L1-ScFab (L-L2-VH-CH1)-L3-ScFab | (GGGGS) 2 | 10 | GGSSGSGSGST GTSSSGTGTSA GTTGTSASTSG SGSGGGGGSGG GGSAGGTATAG ASSGS | 60 | GGSHHGGPE | 9 | 0 |
| DTH2-1 | DT-L1-ScFv (VH-L2-VL) | EASGGPE | 7 | (GGGGS)4 | 20 | | | 1049.773 |
| DTH2-2 | DT-L1-ScFv (VH-L2-VL) | (GGGGS) 2 | 10 | (GGGGS)4 | 20 | | | 1028.027 |
| DTH2-13 | DT-L1-ScFv (VH-L2-VL)-L3-ScFv | EASGGPE | 7 | (GGGGS)4 | 20 | SHGGSLGGS | 9 | 188.444 |
| DTH2-14 | DT-L1-ScFv (VH-L2-VL)-L3-ScFv | (GGGGS) 2 | 10 | (GGGGS)4 | 20 | GGSHHGGPE | 9 | 0 |
| DT22-22 | DT-L1-ScFv (VH-L2-VL)-SS | (GGGGS) 2 | 10 | GSTSGSGKPGS GEGSTKG | 18 | | | 2868 |
| DT22-24 | DT-L1-ScFv (VH-L2-VL)-SS | (GGGGS) 2 | 10 | (GGGGS)4 | 20 | | | 2933 |
| DT22-2 | DT-L1-ScFv (VL-L2-VH) | (GGGGS) 2 | 10 | GSTSGSGKPGS GEGSTKG | 18 | | | 570 |
| DT22-4 | DT-L1-ScFv (VL-L2-VH) | (GGGGS) 2 | 10 | (GGGGS)4 | 20 | | | 1674 |
| DTH2-7 | DT-L1-ScFv (VL-L2-VH) | EASGGPE | 7 | (GGGGS)3 | 15 | | | 1187.860 |
| DTH2-8 | DT-L1-ScFv (VL-L2-VH) | (GGGGS) 2 | 10 | (GGGGS)3 | 15 | | | 450.062 |
| DTH2-44 | DT-L1-ScFv (VL-L2-VH)-L3-CH3 | EASGGPE | 7 | (GGGGS)4 | 20 | GGSGGGGS | 8 | 371.578 |
| DTH2-15 | DT-L1-ScFv (VL-L2-VH)-L3-ScFv | EASGGPE | 7 | (GGGGS)3 | 15 | GGSHHGGS | 8 | 251.416 |
| DTH2-16 | DT-L1-ScFv (VL-L2-VH)-L3-ScFv | (GGGGS) 2 | 10 | (GGGGS)3 | 15 | GGSHHGGPE | 9 | 288.522 |
| DTH2-46 | DT-L1-ScFv (VL-L2-VH)-L3-Tam2 | EASGGPE | 7 | (GGGGS)4 | 20 | GGSGGGGS | 8 | 212.005 |
| DT22-12 | DT-L1-ScFv (VL-L2-VH)-SS | (GGGGS) 2 | 10 | (GGGGS)4 | 20 | | | 1257 |
| DT22-14 | DT-L1-ScFv (VL-L2-VH)-SS | (GGGGS) 2 | 10 | GSTSGSGKPGS GEGSTKG | 18 | | | 3693 |
| PEH2-3 | ScFv (VH-L2-VL)-L1-PE38 | GG | | (GGGGS)3 | 15 | | | 1432 |
| PEH2-4 | ScFv (VH-L2-VL)-L1-PE38 | GGGGS | | (GGGGS)3 | 15 | | | 1404 |
| PEH2-1 | ScFv (VL-L2-VH)-L1-PE38 | GG | | (GGGGS)3 | 15 | | | 2049 |
| PEH2-2 | ScFv (VL-L2-VH)-L1-PE38 | GGGGS | | (GGGGS)3 | 15 | | | 1593 |
| DT22-34 | DT-L1-ScIgG (L-L2-H) | (GGGGS) 2 | 10 | GGSSGSGSGST GTSSSGTGTSA GTTGTSASTSG SGSGGGGSGG GGSAGGTATAG ASSGS | 60 | | | 1514 |
| DTH2-106 | DT-L1-VHH1 | EASGGPE | 7 | | | | | 3791.236 |
| DTH2-108 | DT-L1-VHH1-L3-CH3 | EASGGPE | 7 | | | (GGGGS)2 | 10 | 818.966 |
| DTH2-109 | DT-L1-VHH2 | EASGGPE | 7 | | | | | 4417.456 |
| DTH2-110 | DT-L1-VHH2 | (GGGGS) 2 | 10 | | | | | 3688.224 |
| DTH2-111 | DT-L1-VHH2-L3-CH3 | EASGGPE | 7 | | | (GGGGS)2 | 10 | 5058.481 |

### Example 3: In vitro affinity assay of fusion protein

The IVTT system of DTH2 molecules was incubated in a 24-well plate at 37°C for 3 hours, with 150 µL per molecule.

Recombinant human Her2 protein was diluted from 1 mg/mL to 0.25 µg/mL with coating buffer, added to an ELISA plate at 100 µL/well, and incubated overnight at 4°C.

The plate was washed three times with 300 µL of PBST per wash, followed by addition of 200 µL of blocking buffer, and blocked at 37°C for 1 hour. The plate was washed three times with 300 µL of PBST per wash. The samples were diluted to 20 µg/mL with dilution buffer (PBST containing 1% BSA), followed by 3-fold serial dilution for a total of 11 dilutions. The diluted samples were added to the plate at 100 µL/well, with dilution buffer used as a blank control, and incubated at 37°C for 1 hour.

The plate was washed three times with 300 µL of PBST per wash, followed by addition of HRP-conjugated rabbit anti-human IgG (Fab specific) (1:5000 dilution) at 100 µL/well, and incubated at 37°C for 1 hour.

The plate was washed three times, followed by addition of TMB chromogenic substrate at 100 µL/well, and incubated at 37°C for 10 minutes. The reaction was terminated by addition of stop solution (2 M sulfuric acid) at 50 µL/well, and the absorbance was measured at 450 nm using a microplate reader.

Prism software was used to generate curves by selecting the 4-parameter logistic model, with sample concentration as the x-axis and A450 as the y-axis, and to calculate the affinity EC50 values.

Some of the purified proteins obtained from the previous example were selected for the same EC50 assay. The results are shown in Table 3.

| Molecule ID | Molecular construct | Affinity EC50 (nM) |
|---|---|---|
| DTH2-25 | DT-L1-ScFab (L-L2-VH-CH1)-L3-CH3 | 0.171 |
| DTH2-26 | DT-L1-ScFab (L-L2-VH-CH1)-L3-CH3 | 0.357 |
| DTH2-17 | DT-L1-ScFab (L-L2-VH-CH1)-L3-ScFab (L-L2-VH-CH1) | 0.481 |
| DTH2-28 | DT-L1-Tam2-L3-ScFab (L-L2-VH-CH1) | 0.496 |
| DTH2-4 | DT-L1-ScFab (L-L2-VH-CH1) | 0.669 |
| DTH2-23 | DT-L1-CH3-L3-ScFab (L-L2-VH-CH1) | 0.756 |
| DTH2-3 | DT-L1-ScFab (L-L2-VH-CH1) | 0.930 |
| DTH2-50 | DT-L1-ScFab (L-L2-VH-CH1)-L3-CH3 | 0.630 |
| DTH2-51 | DT-L1-ScFab (L-L2-VH-CH1)-L3-CH3 | 0.590 |
| DTH2-49 | DT-L1-ScFab (L-L2-VH-CH1)-L3-CH3 | 0.740 |
| DTH2-13 | DT-L1-ScFv (VH-L2-VL)-L3-ScFv (VH-L2-VL) | 1.044 |
| DTH2-36 | DT-L1-Tam2-L3-ScFv (VH-L2-VL) | 1.076 |
| DTH2-1 | DT-L1-ScFv (VH-L2-VL) | 1.283 |
| DTH2-111 | DT-L1-VHH2-L3-CH3 | 1.547 |
| DTH2-5 | DT-L1-ScIgG (L-L2-H) | 1.719 |
| DTH2-38 | DT-L1-ScFv (VH-L2-VL)-L3-Tam2 | 1.757 |
| DTH2-16 | DT-L1-ScFv (VL-L2-VH)-L3-ScFv (VL-L2-VH) | 1.988 |
| DTH2-6 | DT-L1-ScIgG (L-L2-H) | 2.331 |
| DTH2-76 | DT-L1-ScFab (L-L2-VH-CH1) | 2.632 |
| DTH2-77 | DT-L1-ScFab (L-L2-VH-CH1) | 2.901 |
| DTH2-81 | DT-L1-ScFab (L-L2-VH-CH1) | 3.023 |
| DTH2-82 | DT-L1-ScFab (L-L2-VH-CH1)-L3-CH3 | 3.163 |
| DTH2-88 | DT-L1-ScFab (L-L2-VH-CH1)-L3-CH3 | 3.414 |
| DTH2-2 | DT-L1-ScFv (VH-L2-VL) | 3.476 |
| DTH2-79 | DT-L1-ScFab (L-L2-VH-CH1) | 3.547 |
| DTH2-15 | DT-L1-ScFv (VL-L2-VH)-L3-ScFv (VL-L2-VH) | 3.567 |
| DTH2-85 | DT-L1-ScFab (L-L2-VH-CH1)-L3-CH3 | 3.932 |
| DTH2-83 | DT-L1-ScFab (L-L2-VH-CH1)-L3-CH3 | 3.976 |
| DTH2-78 | DT-L1-ScFab (L-L2-VH-CH1) | 4.153 |
| DTH2-87 | DT-L1-ScFab (L-L2-VH-CH1)-L3-CH3 | 4.229 |
| DTH2-80 | DT-L1-ScFab (L-L2-VH-CH1) | 4.250 |
| DTH2-7 | DT-L1-ScFv (VL-L2-VH) | 4.567 |
| DTH2-8 | DT-L1-ScFv (VL-L2-VH) | 4.681 |
| DTH2-84 | DT-L1-ScFab (L-L2-VH-CH1)-L3-CH3 | 5.440 |
| DTH2-86 | DT-L1-ScFab (L-L2-VH-CH1)-L3-CH3 | 5.454 |
| DTH2-44 | DT-L1-ScFv (VL-L2-VH)-L3-CH3 | 5.789 |
| DTH2-42 | DT-L1-CH3-L3-ScFv (VL-L2-VH) | 6.590 |

### Example 4: Cytotoxicity assay of fusion protein

(I) The cytotoxicity of the expressed fusion protein was evaluated using the HER2-overexpressing breast cancer cell line SK-BR-3. The cell line is an adherent cell line. After resuspension, the cells were counted and added to a 96-well plate at a density of 3,000 cells per well, with 100 µL of cell culture medium per well, followed by incubation in an incubator overnight at 37°C.

The purified fusion protein was diluted to 30 nM, with the target protein concentration determined based on the fluorescence intensity of eGFP. A 3-fold serial dilution was then performed to generate 10 protein concentration gradients. A 50 µL aliquot of the protein sample was added to 100 µL of cell culture medium, resulting in final protein concentrations ranging from 10 nM to approximately 0.5 pM. A 50 µL aliquot of PBS was added to one well as a negative control. The cell culture medium was incubated in an incubator at 37°C for 72 hours.

The number of viable cells remaining in each well was measured using an MTT assay kit. Viability was calculated by dividing the number of cells in each sample well by the number of cells in the negative control well with PBS. The viability data corresponding to the 10 sample concentrations were fitted using Prism software to obtain the IC50 value of each protein sample.
(1) Cell safety test: The cytotoxicity results of DTH2-4 were used as an example for analysis. ScFab, which does not contain toxin active fragments (DT molecules, toxin molecules), was used to evaluate whether the cytotoxicity against SK-BR-3 was caused by the fusion of DT molecules. DTH2-4 was then tested against Her2-low expressing HEK-293 cells to evaluate the safety and selectivity of the protein.

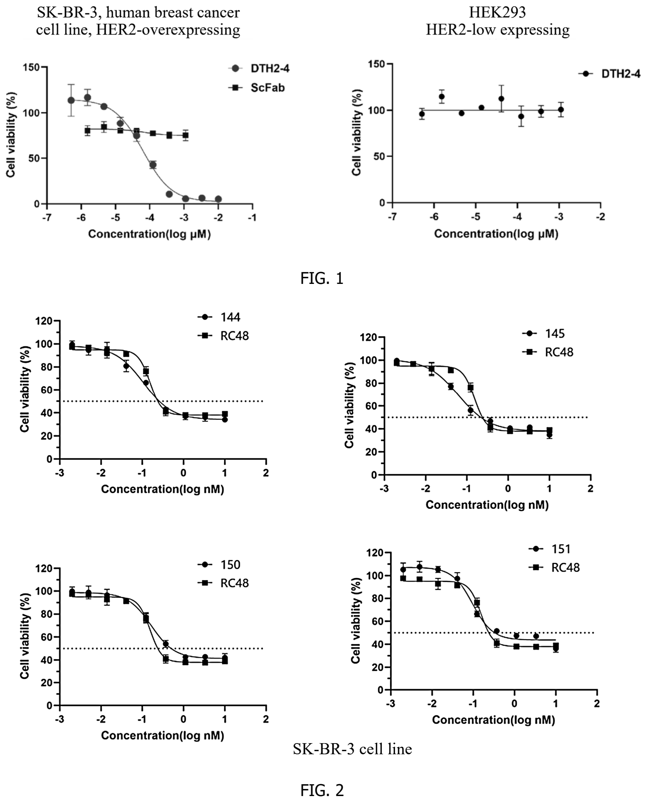

FIG. 1 shows the cytotoxicity assay results of a fusion protein in the example.

As shown in the left panel of FIG. 1, DTH2-4 exhibited an IC50 value of 62.29 pM against SK-BR-3 cells; within the same concentration range, ScFab exhibited no killing effect on SK-BR-3 cells, demonstrating that the cytotoxicity of DTH2-4 was attributed to the presence of the toxin molecule. As shown in the right panel of FIG. 1, within the same concentration range, DTH2-4 exhibited no significant killing effect on HEK-293 cells, demonstrating its safety and usability.

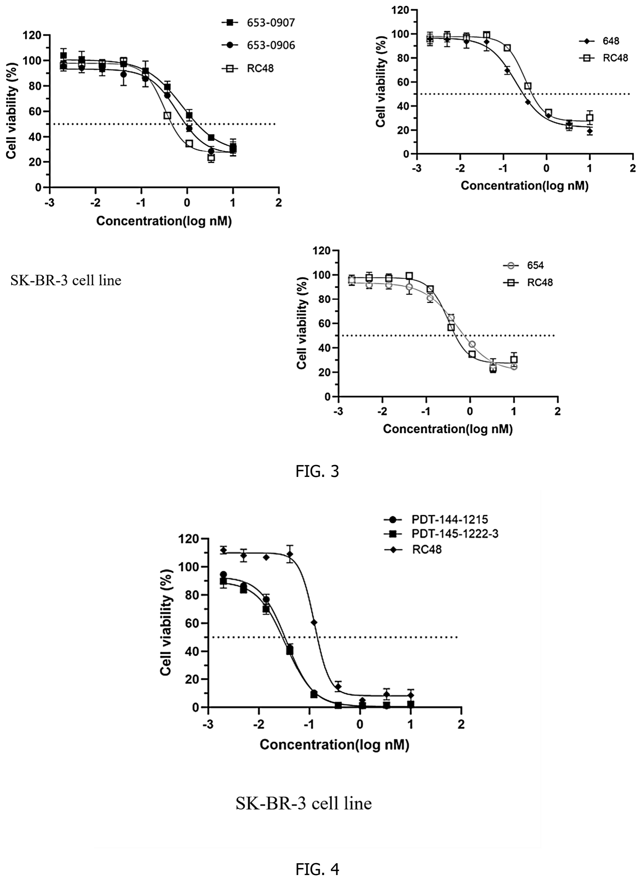

(2) Several fusion proteins with different molecule IDs were tested for their cytotoxicity against the HER2-overexpressing breast cancer cell line SK-BR-3, with a commercially available ADC (RC48) as a control. The results are shown in Table 4 and FIGs. 2 to 4.

| **Table 4** | | | | | |
|---|---|---|---|---|---|
| **Molecule ID** | **DTH2-1** | **DTH2-2** | **DTH2-7** | **DTH2-8** | **RC48** |
| Number corresponding to FIG. 2 | 144 | 145 | 150 | 151 | RC48 |
| IC50 (nM) | 0.1079 | 0.0626 | 0.1647 | 0.09556 | 0.154 |

| **Molecule ID** | **DTH2-109** | **DTH2-109** | **DTH2-104** | **DTH2-110** | **RC48** |
|---|---|---|---|---|---|
| Number corresponding to FIG. 3 | 653 (0906) | 653 (0907) | 648 | 654 | RC48 |
| IC50 (nM) | 0.6084 | 0.7776 | 0.1982 | 0.5203 | 0.3176 |

| **Molecule ID** | **DTH2-1** | **DTH2-2** | | **RC48** | |
|---|---|---|---|---|---|
| Number corresponding to FIG. 4 | PDT-144-1215 | PDT-145-1222-3 | | RC48 | |
| IC50 (nM) | 0.03566 | 0.03379 | | 0.1266 | |

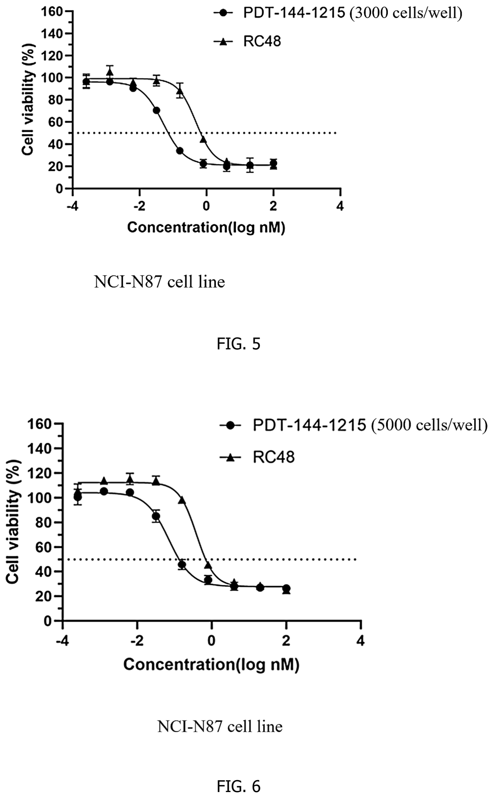

(II) Using the same method, several expressed fusion proteins were further tested for their cytotoxicity against the Her2-overexpressing human gastric cancer cell line NCI-N87. Using the aforementioned method, cells were seeded into a 96-well plate at a density of 1000, 3000, and 5000 cells per well, respectively. The results are shown in Table 5 and FIGs. 5 to 7.

| Table 5 | | |
|---|---|---|
| **Molecule ID** | **DTH2-1** | **RC48** |
| Number corresponding to FIG. 5 | PDT-144-1215 (3000 cells/well) | RC48 |
| IC50 (nM) | 0.05105 | 0.4812 |

| **Molecule ID** | **DTH2-1** | **RC48** |
|---|---|---|
| Number corresponding to FIG. 6 | PDT-144-1215 (5000 cells/well) | RC48 |
| IC50 (nM) | 0.07093 | 0.2275 |

| **Molecule ID** | **DTH2-2 (1000 cells/well)** | **RC48** |
|---|---|---|
| Number corresponding to FIG. 7 | PDT-145-1222-3 | RC48 |
| Bottom | 5.899 | 17.41 |
| Top | 96.7 | 93.17 |
| LogIC50 | -1.651 | -0.5972 |
| HillSlope | -0.9748 | -2.124 |
| IC50 (nM) | 0.02234 | 0.2528 |
| Span | 90.8 | 75.77 |
| R squared | 0.9943 | 0.9924 |

As can be seen from the above, although the EC50 values of several fusion proteins in Table 4 or Table 5 were greater than 1 nM, the cytotoxicity assays demonstrated better efficacy compared to commercially available products, and these fusion proteins can serve as alternatives to currently available therapeutics. Specifically, the corresponding targeting structure and IC50 can be selected as needed to obtain molecules with the desired toxicity.

### Example 4: Animal experiment

Tumor growth inhibition was assessed in a mouse tumor model (ectopic tumor (BT474) in nude mice) using DTH2-1.

Cryopreserved BT474 cells were thawed and cultured in a culture dish at 37°C in a 5% CO₂ incubator. After culturing for a period of time, the cells were passaged and transferred to a new cell culture dish for further cultivation. The cells should undergo at least 2 to 3 passages *in vitro* to ensure the optimal growth status at the time of inoculation. When the cell count and growth status met the experimental requirements, the cells were harvested, resuspended in PBS, and prepared as a sufficient amount of cell suspension for inoculation based on the number of experimental animals.

The mixed cell suspension was aspirated using a 1 mL disposable syringe and slowly injected subcutaneously into the right forelimb axilla of nude mice at approximately 5 × 10⁶ cells per mouse. Following tumor cell inoculation, the long diameter (A) and short diameter (B) of the tumor were measured daily using a vernier caliper after tumor formation in nude mice, and the tumor volume was calculated.

For drug administration, either intratumoral injection or intravenous injection via tail vein was selected. Each nude mouse was injected with medical saline, PC (RC48) 5 mg/kg protein standard, and (DTH2-1) 5 mg/kg protein test article at 2 to 3 injection sites, with 3 nude mice per group.

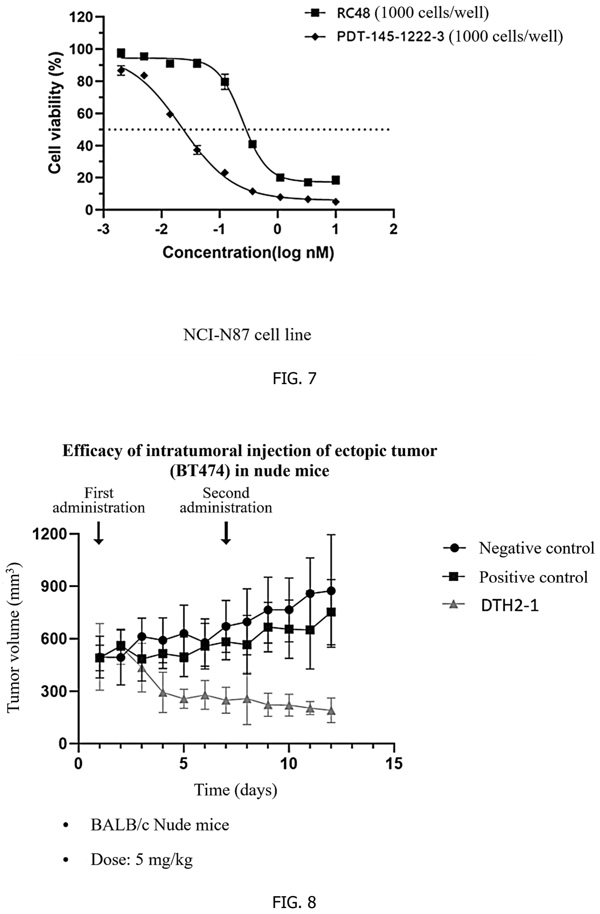

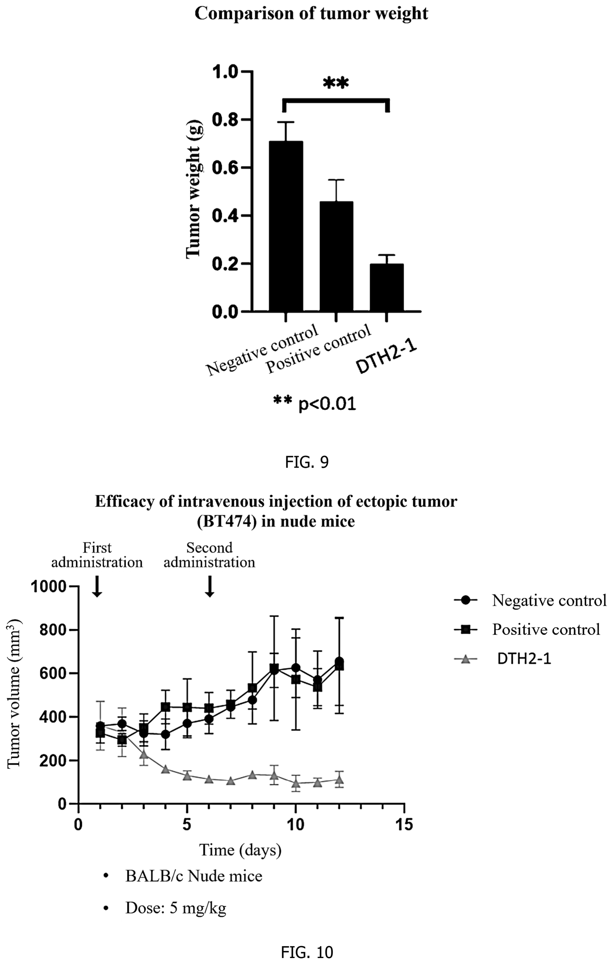

Following drug administration, the long diameter (A) and short diameter (B) of the tumor were measured daily using a vernier caliper, and the tumor volume was calculated (see FIGs. 8 and 10).

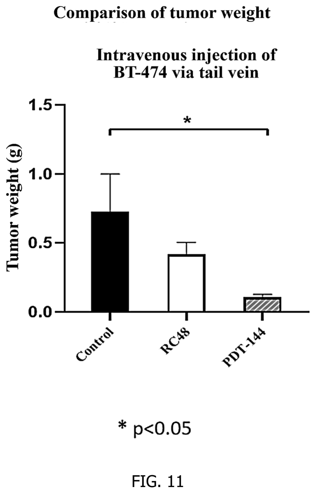

Administration was performed twice, once a week. The experiment was terminated two weeks after the first administration, and the tumor was dissected and weighed (see FIGs. 9 and 11).
Negative control: injection of medical saline;
Positive control: RC48.

The results of intratumoral injection are shown in FIGs. 8 and 9, and the results of intravenous injection via tail vein are shown in FIGs. 10 and 11.

All sequences included herein are summarized in Table 6.

| Table 6 | | |
|---|---|---|
| Sequenc e No. | Name | Sequence |
| SEQ ID NO: 1 | Diphtheria toxin | GADDVVDSSKSFVMENFSSYHGTKPGYVDSIQKGIQKPKSGTQGNYDDD WKGFYSTDNKYDAAGYSVDNENPLSGKAGGVVKVTYPGLTKVLALKVDNA ETIKKELGLSLTEPLMEQVGTEEFIKRFGDGASRVVLSLPFAEGSSSVEYINN WEQAKALSVELEINFETRGKRGQDAMYEYMAQACAGNRVRRSVGSSLSCI NLDWDVIRDKTKTKIESLKEHGPIKNKMSESPNKTVSEEKAKQYLEEFHQT ALEHPELSELKTVTGTNPVFAGANYAAWAVNVAQVIDSETADNLEKTTAAL SILPGIGSVMGIADGAVHHNTEEIVAQSIALSSLMVAQAIPLVGELVDIGFAA YNFVESIINLFQVVHNSYNRPAYSPGHKTQPF |
| SEQ ID NO: 2 | Translocation domain of Diphtheria toxin | SVGSSLSCINLDWDVIRDKTKTKIESLKEHGPIKNKMSESPNKTVSEEKAKQ YLEEFHQTALEHPELSELKTVTGTNPVFAGANYAAWAVNVAQVIDSETADN LEKTTAALSILPGIGSVMGIADGAVHHNTEEIVAQSIALSSLMVAQAIPLVGE LVDIGFAAYNFVESIINLFQVVHNSYNRPAYSPGHKTQP |
| SEQ ID NO: 3 | Catalytic domain of Diphtheria toxin | GADDVVDSSKSFVMENFSSYHGTKPGYVDSIQKGIQKPKSGTQGNYDDD WKGFYSTDNKYDAAGYSVDNENPLSGKAGGVVKVTYPGLTKVLALKVDNA ETIKKELGLSLTEPLMEQVGTEEFIKRFGDGASRVVLSLPFAEGSSSVEYINN WEQAKALSVELEINFETRGKRGQDAMYEYMAQACAGNRVRR |
| SEQ ID NO: 4 | Pseudomonas exotoxin A | PEGGSLAALTAHQACHLPLETFTRHRQPRGWEQLEQCGYPVQRLVALYLAA RLSWNQVDQVIRNALASPGSGGDLGEAIREQPEQARLALTLAAAESERFVR QGTGNDEAGAANGPADSGDALLERNYPTGAEFLGDGGDVSFSTRGTQNW TVERLLQAHRQLEERGYVFVGYHGTFLEAAQSIVFGGVRARSQDLDAIWR GFYIAGDPALAYGYAQDQEPDARGRIRNGALLRVYVPRSSLPGFYRTSLTLA APEAAGEVERLIGHPLPLRLDAITGPEEEGGRLETILGWPLAERTVVIPSAIP TDPRNVGGDLDPSSIPDKEQAISALPDYASQPGKPPREDLK |
| SEQ ID NO: 5 | Translocation domain of Pseudomonas exotoxin A | PEGGSLAALTAHQACHLPLETFTRHRQPRGWEQLEQCGYPVQRLVALYLAA RLSWNQVDQVIRNALASPGSGGDLGEAIREQPEQARLALTLAAAESERFVR QGTGN |
| SEQ ID NO: 6 | Catalytic domain of Pseudomonas exotoxin A | EFLGDGGDISFSTRGTQNWTVERLLQAHRQLEERGYVFVGYHGTFLEAAQ SIVFGGVRARSQDLDAIWRGFYIAGDPALAYGYAQDQEPDARGRIRNGALL RVYVPRSSLPGFYRTGLTLAAPEAAGEVERLIGHPLPLRLDAITGPEEEGGRL ETILGWPLAERTVVIPSAIPTDPRNVGGDLDPSSIPDKEQAISALPDYASQP GKPPREDLK |
| SEQ ID NO: 7 | Cholerae toxin | VEDELNIFDECRSPCSLTPEPGKPIQSKLSIPSDVVLDEGVLYYSMTINDEQ NDIKDEDKGESIITIGEFATVRATRHYVNQDAPFGVIHLDITTENGTKTYSY NRKEGEFAINWLVPIGEDSPASIKISVDELDQQRNIIEVPKLYSIDLDNQTLE QWKTQGNVSFSVTRPEHNIAISWPSVSYKAAQKEGSRHKRWAHWHTGLA LCWLVPMDAIYNYITQQNCTLGDNWFGGSYETVAGTPKVITVKQGIEQKP VEQRIHFSKGNAMSALAAHRVCGVPLETLARSRKPRDLTDDLSCAYQAQNI VSLFVATRILFSHLDSVFTLNLDEQEPEVAERLSDLRRINENNPGMVTQVLT VARQIYNDYVTHHPGLTPEQTSAGAQAADILSLFCPDADKSCVASNNDQA NINIESRSGRSYLPENRAVITPQGVTNWTYQELEATHQALTREGYVFVGYH |
| | | GTNHVAAQTIVNRIAPVPRGNNTENEEKWGGLYVATHAEVAHGYARIKEG TGEYGLPTRAERDARGVMLRVYIPRASLERFYRTNTPLENAEEHITQVIGHS LPLRNEAFTGPESAGGEDETVIGWDMAIHAVAIPSTIPGNAYEELAIDEEAV AKEQSISTKPPYKERKDELK |
| SEQ ID NO: 8 | Translocation domain of Cholerae toxin | VEDELNIFDECRSPCSLTPEPGKPIQSKLSIPSDVVLDEGVLYYSMTINDEQ NDIKDEDKGESIITIGEFATVRATRHYVNQDAPFGVIHLDITTENGTKTYSY NRKEGEFAINWLVPIGEDSPASIKISVDELDQQRNIIEVPKLYSIDLDNQTLE QWKTQGNVSFSVTRPEHNIAISWPSVSYKAAQKEGSRHKRWAHWHTGLA LCWLVPMDAIYNYITQQNCTLGDNWFGGSYETVAGTPKVITVKQGIEQKP VEQRIHFSKGNAMSALAAHRVCGVPLETLARSRKPRDLTDDLSCAYQAQNI VSLFVATRILFSHLDSVFTLNLDEQEPEVAERLSDLRRINENNPGMVTQVLT VARQIYNDYVTHHPGLTPEQTSAGAQAADILSLFCPDADKSCVASNNDQA NINIESRSGRSYLPENR |
| SEQ ID NO: 9 | Catalytic domain of Cholerae toxin | AVITPQGVTNWTYQELEATHQALTREGYVFVGYHGTNHVAAQTIVNRIAP VPRGNNTENEEKWGGLYVATHAEVAHGYARIKEGTGEYGLPTRAERDARG VMLRVYIPRASLERFYRTNTPLENAEEHITQVIGHSLPLRNEAFTGPESAGG EDETVIGWDMAIHAVAIPSTIPGNAYEELAIDEEAVAKEQSISTKPPYKERK DELK |
| SEQ ID NO: 10 | Linker | (GGGGS)2 |
| SEQ ID NO: 11 | Linker | EASGGPE |
| SEQ ID NO: 12 | Linker | GGSSGSGSGSTGTSSSGTGTSAGTTGTSASTSGSGSGGGGGSGGGGSAG GTATAGASSGS |
| SEQ ID NO: 13 | Linker | GAGAGSGAGAGSGAGAGSGAGAGSGAGAGSGAGAGS |
| SEQ ID NO: 14 | Linker | (GGGGS)3 |
| SEQ ID NO: 15 | Linker | GS |
| SEQ ID NO: 16 | Linker | KESGSVSSEQLA |
| SEQ ID NO: 17 | Heavy chain H (CD22) | EVQLVQSGAEVKKPGASVKVSCKASGYRFTNYWIHWVRQAPGQGLEWIG GINPGNNYATYRRKFQGRVTMTADTSTSTVYMELSSLRSEDTAVYYCTREG YGNYGAWFAYWGQGTLVTVSSASTKGPSVFPLAPCSRSTSESTAALGCLVK DYFPEPVTVSWNSGALTSGVHTFPAVLQSSGLYSLSSVVTVPSSSLGTKTYT CNVDHKPSNTKVDKRVESKYGPPCPPCPAPEFLGGPSVFLFPPKPKDTLMIS RTPEVTCVVVDVSQEDPEVQFNWYVDGVEVHNAKTKPREEQFNSTYRVVS VLTVLHQDWLNGKEYKCKVSNKGLPSSIEKTISKAKGQPREPQVYTLPPSQ EEMTKNQVSLTCLVKGFYPSDIAVEWESNGQPENNYKTTPPVLDSDGSFFL YSRLTVDKSRWQEGNVFSCSVMHEALHNHYTQKSLSLSLGK |
| SEQ ID NO: 18 | Linker | EGKSSGSGSES |
| SEQ ID NO: 19 | Linker | GSAPAPAGS |
| SEQ ID NO: 20 | Linker | GGGGS |
| SEQ ID NO: 21 | Linker | GGS |
| SEQ ID NO: 22 | Linker | GGSGGGGS |
| SEQ ID NO: 23 | Linker | GGSHHGHKTQPFGGGGSGGGGS |
| SEQ ID NO: 24 | Linker | GGSHHTQPFGGGGSGGGGS |
| SEQ ID NO: 25 | Linker | GGSHHQPFEASGGPE |
| SEQ ID NO: 26 | Linker | GGSHHEASGGPE |
| SEQ ID NO: 27 | Linker | GGSHHGGPE |
| SEQ ID NO: 28 | Linker | (GGGGS)4 |
| SEQ ID NO: 29 | Linker | SHGGSLGGS |
| SEQ ID NO: 30 | Linker | GGSHHGGS |
| SEQ ID NO: 31 | Linker | KESGSVSSEQLA |
| SEQ ID NO: 32 | Light chain L (Her2) | DIQMTQSPSSLSASVGDRVTITCRASQDVNTAVAWYQQKPGKAPKLLIYSA SFLYSGVPSRFSGSRSGTDFTLTISSLQPEDFATYYCQQHYTTPPTFGQGTK VEIKRTVAAPSVFIFPPSDEQLKSGTASVVCLLNNFYPREAKVQWKVDNAL QSGNSQESVTEQDSKDSTYSLSSTLTLSKADYEKHKVYACEVTHQGLSSPV TKSFNRGEC |
| SEQ ID NO: 33 | Variable domain VH (Her2) | EVQLVESGGGLVQPGGSLRLSCAASGFNIKDTYIHWVRQAPGKGLEWVAR IYPTNGYTRYADSVKGRFTISADTSKNTAYLQMNSLRAEDTAVYYCSRWGG DGFYAMDYWGOGTLVTVSS |
| SEQ ID NO: 34 | First constant domain CH1 (Her2) | ASTKGPSVFPLAPSSKSTSGGTAALGCLVKDYFPEPVTVSWNSGALTSGVH TFPAVLQSSGLYSLSSVVTVPSSSLGTQTYICNVNHKPSNTKVDKKVEPKSC DK |
| SEQ ID NO: 35 | Variable domain VL (Her2) | EVQLVESGGGSDIQMTQSPSSLSASVGDRVTITCRASQDVNTAVAWYQQK PGKAPKLLIYSASFLYSGVPSRFSGSRSGTDFTLTISSLQPEDFATYYCQQHY TTPPTFGQGTKVEIK |
| SEQ ID NO: 36 | Heavy chain H (Her2) | EVQLVESGGGLVQPGGSLRLSCAASGFNIKDTYIHWVRQAPGKGLEWVAR IYPTNGYTRYADSVKGRFTISADTSKNTAYLQMNSLRAEDTAVYYCSRWGG DGFYAMDYWGQGTLVTVSSASTKGPSVFPLAPSSKSTSGGTAALGCLVKDY |
| | | FPEPVTVSWNSGALTSGVHTFPAVLQSSGLYSLSSVVTVPSSSLGTQTYICN VNHKPSNTKVDKKVEPKSCDK |
| SEQ ID NO: 37 | Third constant domain CH3 (Her2) | GQPREPQVYTLPPSREEMTKNQVSLTCLVKGFYPSDIAVEWESNGQPENN YKTTPPVLDSDGSFFLYSKLTVDKSRWQQGNVFSCSVMHEALHNHYTQKS LSLSPGK |
| SEQ ID NO: 38 | Tamavidins2 | SDVQSSLTGTWYNELNSKMELTANKDGTLTGKYLSKVGDVYVPYPLSGRYN LQPPAGQGVALGWAVSWENSKIHSATTWSGQFFSESSPVILTQWLLSSSTA RGDVWESTLVGNDSFTKTAPTEQQIAHAQLHCRAPRLK |
| SEQ ID NO: 39 | Primer 1 | CTGGTGGTGGTGGTTCTGACATCCAAATGACCCAATCTCCATCTTC |
| SEQ ID NO: 40 | Primer 2 | GTGGTGGTGAGAACCACCCTTGTCACAAGACTTTGGTTCAACCTTC |
| SEQ ID NO: 41 | Primer 3 | GAACCAAAGTCTTGTGACAAGGGTGGTTCTCACCACCACCAC |
| SEQ ID NO: 42 | Primer 4 | GGAGATTGGGTCATTTGGATGTCAGAACCACCACCACCAGAACC |
| SEQ ID NO: 43 | Linker | GG |
| SEQ ID NO: 44 | Linker | GSAEAAAKEAAAKAGGGGS |
| SEQ ID NO: 45 | Linker | GGSSGSGSGSTGTSSSGTGTSAGTTGTSASTSGSGSGGGGGSGGGGSAG GTATAGASSGS |
| SEQ ID NO: 46 | eGFP | VSKGEELFTGVVPILVELDGDVNGHKFSVRGEGEGDATNGKLTLKFICTTGK LPVPWPTLVTTLTYGVQCFSRYPDHMKQHDFFKSAMPEGYVQERTISFKD DGTYKTRAEVKFEGDTLVNRIELKGIDFKEDGNILGHKLEYNFNSHNVYITA DKQKNGIKANFKIRHNVEDGSVQLADHYQQNTPIGDGPVLLPDNHYLSTQ SKLSKDPNEKRDHMVLLEFVTAAGITLGMDELYK |
| SEQ ID NO: 47 | Linker | GSTSGSGKPGSGEGSTKG |
| SEQ ID NO: 48 | Linker | GSAGSAAGSGEF |
| SEQ ID NO: 49 | Linker | GSAPAPAGGGGS |
| SEQ ID NO: 50 | Linker | KESGSVSSEQLA |
| SEQ ID NO: 51 | Linker | CPPCPAPELL |
| SEQ ID NO: 52 | VHH1 (Her2) | EVQLQESGGGLVQPGGSLRLSCAASGFIFSNDAMTWVRQAPGKGLEWVSS INWSGTHTNYADSVKGRFTISRDNAKRTLYLQMNSLKDEDTALYYCVTGY GVTKTPTGQGTQVTVSS |
| SEQ ID NO: 53 | VHH2 (Her2) | EVQLVESGGGLVQAGGSLRLSCAASGITFSINTMGWYRQAPGKQRELVALI SSIGDTYYADSVKGRFTISRDNAKNTVYLQMNSLKPEDTAVYYCKRFRTAA QGTDYWGQGTQVTVSS |
| SEQ ID NO: 54 | Variable domain VL (CD22) | DVQVTQSPSSLSASVGDRVTITCRSSQSLANSYGNTFLSWYLHKPGKAPQL LIYGISNRFSGVPDRFSGSGSGTDFTLTISSLQPEDFATYYCLQGTHQPYTF GQGTKVEIKR |
| SEQ ID NO: 55 | Variable domain VH (CD22) | EVQLVQSGAEVKKPGASVKVSCKASGYRFTNYWIHWVRQAPGQGLEWIG GINPGNNYATYRRKFQGRVTMTADTSTSTVYMELSSLRSEDTAVYYCTREG YGNYGAWFAYWGQGTLVTVSS |
| SEQ ID NO: 56 | Light chain L (CD22) | DVQVTQSPSSLSASVGDRVTITCRSSQSLANSYGNTFLSWYLHKPGKAPQL LIYGISNRFSGVPDRFSGSGSGTDFTLTISSLQPEDFATYYCLQGTHQPYTF GQGTKVEIKRTVAAPSVFIFPPSDEQLKSGTASVVCLLNNFYPREAKVQWK VDNALQSGNSQESVTEQDSKDSTYSLSSTLTLSKADYEKHKVYACEVTHQ GLSSPVTKSFNRGEC |
| SEQ ID NO: 57 | CH1 (CD22) | ASTKGPSVFPLAPCSRSTSESTAALGCLVKDYFPEPVTVSWNSGALTSGVHT FPAVLQSSGLYSLSSVVTVPSSSLGTKTYTCNVDHKPSNTKVDKRVES |
| SEQ ID NO: 58 | 8His | HHHHHHHH |
| SEQ ID NO: 59 | Dph1p (DPH1 protein) | MTMGEVEKKPRRRFVGIKKAAEESDPSGSTTELVKKIKPKTNIRHAINQIPP ELLNDEELNAAIKLLPSNYNFEIHKTVWNIRKHKAKRVAIQMPEGLLIYSLVI SDILEQFCQCEIVVMGDVSYGACCIDDYTARSLDCDFIVHYAHSCLVPIDIT TIKVLYIFVTINIDETHLIKTLQKNFPRGSRIAAFGTIQFNPTIHSMKDKLLQS EEHMLYIIPPQIKPLSKGEVLGCTSERLNKEQIDAMVYVGDGRFHLESAMIH NPEIPAFRYDPYNRRFTRERYDQKQLVEVRASAIDKARSSKKVGLILGALGR QGNLATVENLETKLKASGRTVVKIILSEIFPQKLAMFDDIDAFVQVACPRLSI DWGYAFNKPLLTPYETNVMLENDRMFNEKYYPMDYYHINGYGRGKVPNH DDVTI |
| SEQ ID NO: 60 | Dph2p (DPH2 protein) | MNDSVLVAPSLSTAQTEDTFEFQSYGETEHSRSYLGSDVTKDNLVELVSAY YSVPELIQYFEEHPQYKKITLQFPDELVLDSSIVVQLMQQELVKAEDETSQS FNTIDTDEVLHNEKSCGNCSGCDCSSKIDKTETKRKVWILADTSYSSCCVD EVASEHVKGDIVVHFGDACMNAVQKLPVVYSLGRPVLDLDLVVSQFKLKYA AKDQKICLMADAPHSMHMKSIYDILHDKEGYKNVVYSDINQDMLQSDTHI VGYNNAVEHDERLRKCVTCGNRNIYAEVDVSELNEYDLFHVTIPKDPHLLY LTTKFQSVTLYDPSSGMLNEGPFPSMMKRYKFMHMARTAGTIGILVNTLSL RNTKETMNKLTKLLKENGKKHYLFVVGKPNVAKLANFEPIDIWCILGCGQG GIVLDQYNEFYKPIITPYELLMALSDEVTWTGQWITDFKSIINQIENEVNDS DDMEQDILSSGTECRSDEDEAPEFNAVTGKYVSTSRPLRQIARLEIETPVEE VRASDSTELVKQFSQTVAIRNTVSTSAAFLQTRHWTGLGSDYKDDEDGEE DGATVEEGTAGVARSYQFDELNKKT |
| SEQ ID NO: 61 | Dph3p (DPH3 protein) | MAEELLNKHDPKLAYVVKYNHDSPNPRELASPLEMKRVARPLRHIHYVPK SLIFYSKNHPPQFSYETKIKTPISKDVISVQVEYVGLNPVDLKIFNSYTRHM NYEVGIGREYYGKISEVGSNLADTWSVGDEVCGTFWHPNLGKGTCESTIL VDPKVDVVLPKPVNISTQQAASTFYCLGAAFNILDKLDQEGKLNQSSNILIN GASTMTGIFALQLLKFHYRILNRIVLISSTSGMNFIKSNLPELVDELLFIDYAA TNGKIYKPLSDLVASNELVEYNNETGNFITQPFGQGSFNLVLDFIGGYDIIG HSSSILSKDATYITTVGDNKSNYKKDTFNSWGTASSNIRKLFGQVLWSFDY |
| | | QMFYFDPNAKLATNEWPKKCHELIESEVVKFIPVDRVYDWKEHQKAFSYL QTGRAHGKVVLKVEKF |
| SEQ ID NO: 62 | Dph4p (DPH4 protein) | MQRSFYEILGIESDASNIEIKRAYRAMLLETHPDKSGLNYNSNGNNTSSHS VTDIQEAYQTLIDQDLRKKYDEELAESFKKLGFHNAGDGLDLFSLDLFDYS GDEQSFSMNCPRCQITDGFQLTEDALEEHAIDYEGGGYFVLVQCSACSLW LKVLFDVMEE |
| SEQ ID NO: 63 | Dph5p (DPH5 protein) | MLYFIGLGLSYETDITVRGLNAIKQCSRVYLEHYTSILMTASLEELEEFYGKK VTLADRELVESGAEELLRDADKEDVAFLVVGDVFGATTHTDLVLRAKQRNIP VEVIHNASVMNAVGSCGLQLYNFGQTISMVFFTDSWRPDSWYDKIWENR KIGLHTLVLLDIKVKEQSIENMARGRLIYEPPRYMSIAQCCEQLLEIEETRGT EAYTPDTPCVAISRLGSASQTFKAGTIKELAEYDSGEPLHSLVILGRQTHELE IEYLLEFCDDREKFKQDVASDQEYFKPPAWVPPPEDED |
| SEQ ID NO: 64 | Dph6p (DPH6 protein) | MKFVALISGGKDSNYNILHCLKQDHELIAFANLHPENEDEQELDSFMFQTV GHDLIRWYPECSGVPLYRQALHKNGSKNIALNYTETKDDEIEDLYKLLRKIQ LDSPDLEAVSVGAILSSYQRTRVEDVCSRLGLTTLSYLWQRDQRELMQEMC SMSKDTIVTTDDCDNVGKLDARIIKVAAIGLNQNHLGKSLPEILPTMLKLNS LYDVHICGEGGEFETMVLDTPFFKHGHLQLNSINDVTDTSNDGVFAATFNV EYVPEALSPTKLEKELEKLPVPAVLNEKWYEMYLRLMTIDLKKLNNCPNHH NVSPSVPVSINEVGKLLYISNIAPSKGESLKEKCLDVFSQLTSILSSRSIFACQ ILSSSLLLSDMNNFQEVNSYYNEYFNVTKIGPLPPARACVESSFLKHPVQLS VVVDLSADCVPTDNGIILNRSKDGLHVQGRSYWCPCNIGPYSQATWNQS DRNKVTYISGQIGLEPSSMKLWGDTTLLENPDIAEVVLSLRNYFTLSETVNS SIPLTMVCYISQSYVLPAVRSAWSLFAKELAEESELWFDQEPVGVDSLVIVK VSNLPKNALCEWTGMNCQNLAIEDDYDEDDLAAQIHMNLTLKSQTDIELP NYAHDVIISENGFKRHFITLFLDSHEQLILTLSECKNAQITLFFSKDYAVPEY THVEYIPVEQVYDKSSPRAYGLVIKY |
| SEQ ID NO: 65 | Dph7p (DPH7 protein) | MAQQSLQKHPHDEDAEYDSNIGIAYHLRGATKLEVKSFCRMEETAADKRV TTRLPPCCLCILREKFIIVGTYLLHKDSGNRTGSIEVYDKELNLLSSIKTYGAI LDLKLNPFDDTLLATAHSTGNMILWTISVDDDNSSIDIKLLNNLQVFDPTCL ITSLQFSPLLDNLVLLTTTNGDFATVDIIHGEICFIEEVEPSYFDSSTINYIEVP NADIKVINVQNDHEQIFHFKHSLECWTGEFGCLAPLKDVVFTGGDDSAIAA HDLNSGKKIWSNSKIHQAGVTAIKAARETFRGNKPTSLITGSYDDHIRSFD LRMLSETTIYPGTDVPVVNKWEDNLQGGVWRFSEAPTNDGLSNNRLMVC CMYNGAKVVNVQNDHFVTEQFIKEGHESMCYGGDWSKETVATCSFYDKS LQLWKSPN |
| SEQ ID NO: 66 | eEF2 | MVAFTVDQIRSLMDKVTNVRNMSVIAHVDHGKSTLTDSLVQRAGIISAAKA GEARFTDTRKDEQERGITIKSTAISLFSEMSDDDVKDIKQKTDGNAFLINLI DSPGHVDFSSEVTAALRVTDGALVVVDTVEGVCVQTETVLRQSLAERIKPV VVINKVDRALLELQVSKEDLYQSFSRTVESVNVIISTYADEVLGDVQVYPQR GTVAFGSGLHGWAFTVRQFANRYSKKFGVDREKMMDRLWGDSYFNPKTK KWTNKERDADGKPLERAFNMFVLDPIFRLFAAIMNFKKEEIPVLLEKLEINL KGDEKELEGKNLLKVVMRKFLPAADALLEMIILHLPSPVTAQNYRAEQLYEG PSDDPACIAIKNCDPKSDLMLYVSKMVPTSDKGRFYAFGRVFAGTVKSGQK VRIQGPNFIPGKKEDLFIKAIQRAVLMMGRFVEPIDDCPAGNIIGLVGIDQF |
| | | LLKTGTLTTFEGAHNMKVMKFSVSPVVQVAVEVKNANDLPKLVEGLKRLSK SDPCVLVSMSESGEHIVAGTGELHLEICLQDLENDHAGIPLKISPPVVAYRE TVEGESSQTALSKSPNKHNRIYLKAQPIDEEVSLAIEGGKINPRDDFKARAR IMADEFGWDVTDARKIWCFGPDGNGPNLVVDQTKAVQYLNEIKDSVVAAF QWATKEGPIFGEQMRSVRVNILDVTLHADAIHRGGGQIIPTMRRATYAGFL LAEPKIQEPVFLVEIQCPEQAIGGIYSVLNKKRGQVVSEEQRPGTPLFTVKA YLPINESFGFTGELRQATGGQAFPQMVFDHWATLGTDPLDPSTKAGEIVLA ARKRQGMKEEVPGWQEYYDKL |

## Claims

1. A fusion protein, comprising:
an effector moiety A for exerting killing effect on a target cell, comprising a toxin molecule;
a targeting vector moiety B that binds to a target site on the target cell, wherein the targeting vector moiety is derived from an antibody or a cytokine;
a first linker L1 for linking the effector moiety A to the targeting vector moiety B,
wherein the first linker L1 comprises at least 3 amino acid residues, preferably 3 to 20 amino acid residues, and more preferably 3 to 10 amino acid residues;
the effector moiety A, the first linker L1, and the targeting vector moiety B are connected in an orientation of A-L1-B from the N-terminus to the C-terminus or in an orientation of B-L1-A from the N-terminus to the C-terminus.

2. The fusion protein according to claim 1, wherein
the toxin molecule is any one of the following toxic polypeptides: (a) a plant-derived toxin; (b) a bacterial-derived toxin; (c) a fungal-derived toxin; (d) a human-derived protein toxin; (e) a functional fragment of any one of (a) to (d); (f) a functional fragment derived from any one of (a) to (d) or a variant of the functional fragment;
preferably, the toxin molecule is derived from: Diphtheria toxin, Pseudomonas exotoxin A, or Cholerae toxin.

3. The fusion protein according to claim 1 or 2, wherein
the structure of the toxin molecule comprised in the effector moiety is any one of the following:
(1) a translocation domain DT and a catalytic domain DA of the Diphtheria toxin, preferably connected in an orientation of DA-DT from the N-terminus to the C-terminus;
(2) the catalytic domain DA of the Diphtheria toxin;
(3) a translocation domain PT and a catalytic domain PA of the Pseudomonas exotoxin A, preferably connected in an orientation of PT-PA from the N-terminus to the C-terminus;
(4) the catalytic domain PA of the Pseudomonas exotoxin A;
(5) a translocation domain CT and a catalytic domain CA of the Cholerae toxin, preferably connected in an orientation of CT-CA from the N-terminus to the C-terminus;
(6) the catalytic domain CA of the Cholerae toxin.

4. The fusion protein according to claim 3, wherein
the toxin molecule has an amino acid sequence shown in any one of SEQ ID NOs: 1 to 9, or has an amino acid sequence with at least 80%, 85%, 90%, 95%, or 99% sequence identity to any one of SEQ ID NOs: 1 to 9.

5. The fusion protein according to claim 1 or 2, wherein
the targeting vector moiety is an antibody, wherein the antibody is any one or more of a monoclonal antibody, a chimeric antibody, a reshaped antibody, a small molecule antibody, a multispecific antibody, and a multivalent antibody;
preferably, the antibody is any one or more of a small molecule antibody or a multivalent antibody;
more preferably, the small molecule antibody is any one or more of Fv, Fab, ScFv, ScFab, ScIgG, dsFv, a nanobody, and a single-domain antibody;
the multivalent antibody is a tandem antibody formed by at least two single-chain antibodies in tandem and an antibody polymeric fusion protein formed by a small molecule antibody and a polymeric protein; further, the antibody polymeric fusion protein is a dimer or a tetramer.

6. The fusion protein according to claim 5, wherein
the antibody is ScFab, ScFv, ScIgG, a nanobody, two ScFabs in tandem, two ScFvs in tandem, two nanobodies in tandem, a fusion of ScFab with an antibody fragment CH3, a fusion of ScFv with an antibody fragment CH3, two nanobodies fused in tandem via CH3, a fusion of ScFab with Tamavidins2 protein, or a fusion of ScFv with Tamavidins2 protein.

7. The fusion protein according to claim 6, wherein
the structure of ScFab satisfies the following: the light chain portion and the heavy chain portion of ScFab are connected in an orientation from the N-terminus to the C-terminus, or the heavy chain portion and the light chain portion of ScFab are connected in an orientation from the N-terminus to the C-terminus;
ScFab and the antibody fragment CH3 are connected in an orientation from the N-terminus to the C-terminus or from the C-terminus to the N-terminus;
the heavy chain portion and the light chain portion of ScFv are connected in an orientation from the N-terminus to the C-terminus;
the fusion of ScFab with Tamavidins2 protein is connected in an orientation from the N-terminus to the C-terminus as Tamavidins2 protein to ScFab;
the fusion of ScFv with Tamavidins2 protein is connected in an orientation from the N-terminus to the C-terminus as Tamavidins2 protein to ScFv.

8. The fusion protein according to claim 6 or 7, wherein
the light chain portion and the heavy chain portion of each of ScFab, ScFv, ScFv, and ScIgG are connected via a second linker L2;
the second linker L2 comprises at least 15 amino acid residues, preferably 15 to 180, 15 to 120, 15 to 90, 15 to 65, or 2060 amino acid residues.

9. The fusion protein according to claim 7, wherein
the second linker L2 comprises any one or more of predominant amino acid residues of G, S, T, and A, and the number of the predominant amino acid residues accounts for at least more than 50%, 60%, 70%, 80%, or 90% of the total number of amino acid residues in the second linker; more preferably, the second linker L2 comprises any one or more of G and S among the predominant amino acid residues, and the total number of the predominant amino acid residues accounts for at least more than 40%, or more than 45%, or more than 60%, or more than 70%, or more than 80% of the total number of amino acid residues in the second linker;
further, the second linker L2 has an amino acid sequence shown in any one of SEQ ID NOs: 10, 12, 13, 14, 17, 28, 45, and 47, or has an amino acid sequence with at least 80%, 85%, 90%, 95%, or 99% sequence identity to any one of SEQ ID NOs: 10, 10, 12, 13, 14, 17, 28, 45, and 47.

10. The fusion protein according to any one of claims 5 to 9, wherein
two single-chain antibodies, or a small molecule antibody and a polymeric protein, are connected via a third linker L3;
the third linker L3 is absent or comprises at least 3 amino acid residues; preferably, the third linker L3 comprises 5 to 20 amino acid residues; preferably, the third linker L3 comprises 8 to 20 amino acid residues, or 8 to 15 amino acid residues, or 8 to 12 amino acid residues; more preferably, the third linker L3 comprises two predominant amino acid residues, G and S, and the number of the predominant amino acid residues accounts for more than 40% of the total number of amino acid residues in the third linker;
for example, the third linker L3 has an amino acid sequence shown in any one of SEQ ID NOs: 10, 14, 18, 20 to 30, 44, and 48 to 51, or has an amino acid sequence with at least 80%, 85%, 90%, 95%, or 99% sequence identity to any one of SEQ ID NOs: 10, 14, 18, 20 to 30, 44, and 48 to 51.

11. The fusion protein according to any one of claims 1 to 7, wherein
the antibody is an antibody that specifically binds to any one or more of Her2 and CD22.

12. The fusion protein according to claim 11, wherein
the antibody is derived from IgM, IgG, IgA, IgD, and IgE;
preferably, the antibody is derived from Trastuzumab, Rituximab, Bevacizumab, Adalimumab, Pembrolizumab, Ustekinumab, Nivolumab, Ocrelizumab, Palivizumab, Infliximab, Omalizumab, Envafolimab, Atlizumab, Atezolizumab, or Cetuximab, or avariants thereof.

13. The fusion protein according to any one of claims 5 to 12, wherein
for Fab or ScFab:
the light chain portion constituting the Fab or ScFab has an amino acid sequence shown in SEQ ID NO: 32, or has an amino acid sequence with at least 80%, 85%, 90%, 95%, or 99% sequence identity to SEQ ID NO: 32; or
the variable domain of the heavy chain portion constituting the Fab or ScFab has an amino acid sequence shown in SEQ ID NO: 33 or 55, or has an amino acid sequence with at least 80%, 85%, 90%, 95%, or 99% sequence identity to SEQ ID NO: 33 or 55; or
the first constant domain CH1 of the heavy chain portion constituting the Fab or ScFab has an amino acid sequence shown in SEQ ID NO: 34 or 57, or has an amino acid sequence with at least 80%, 85%, 90%, 95%, or 99% sequence identity to SEQ ID NO: 34 or 57;
for Fv, dsFv, or ScFv:
the variable domain of the light chain portion constituting the Fv, dsFv, or ScFv has an amino acid sequence shown in SEQ ID NO: 35 or 54, or has an amino acid sequence with at least 80%, 85%, 90%, 95%, or 99% sequence identity to SEQ ID NO: 35 or 54; or
the variable domain of the heavy chain portion constituting the Fv, dsFv, or ScFv has an amino acid sequence shown in SEQ ID NO: 33 or 55, or has an amino acid sequence with at least 80%, 85%, 90%, 95%, or 99% sequence identity to SEQ ID NO: 33 or 55;
for ScIgG:
the light chain portion constituting the ScIgG has an amino acid sequence shown in SEQ ID NO: 32 or 56, or has an amino acid sequence with at least 80%, 85%, 90%, 95%, or 99% sequence identity to SEQ ID NO: 32 or 56; or
the heavy chain portion constituting the ScIgG has an amino acid sequence shown in SEQ ID NO: 36 or 17, or has an amino acid sequence with at least 80%, 85%, 90%, 95%, or 99% sequence identity to SEQ ID NO: 36 or 17;
for the antibody fragment CH3 involved, the antibody fragment CH3 has the amino acid sequence shown in SEQ ID NO: 37, or has an amino acid sequence with at least 80%, 85%, 90%, 95%, or 99% sequence identity to SEQ ID NO: 37;
for the Tamavidins2 protein involved, the Tamavidins2 protein has the amino acid sequence shown in SEQ ID NO: 38, or has an amino acid sequence with at least 80%, 85%, 90%, 95%, or 99% sequence identity to SEQ ID NO: 38;
for the nanobody involved, the nanobody has an amino acid sequence shown in SEQ ID NO: 52 or 53, or has an amino acid sequence with at least 80%, 85%, 90%, 95%, or 99% sequence identity to SEQ ID NO: 52 or 53.

14. The fusion protein according to any one of claims 1 to 13, wherein
the fusion protein is suitable for cell-free *in vitro* synthesis; preferably, the cell-free *in vitro* synthesis involves: in a cell-free *in vitro* synthesis system, using a coding nucleic acid of the fusion protein as a DNA template or RNA template, incubating under certain conditions for a certain period of time to obtain the fusion protein.

15. The fusion protein according to claim 14, wherein
the cell-free *in vitro* synthesis system comprises a cell extract, wherein the cell extract is derived from a yeast strain;
further, the yeast strain is *Saccharomyces cerevisiae*, *Kluyveromyces*, or a combination thereof; in another preferred example, the *Kluyveromyces* is *Kluyveromyces lactis, Kluyveromyces marxianus*, *Kluyveromyces dobzhanskii*, or a combination thereof.

16. The fusion protein according to claim 15, wherein
the yeast strain is genetically engineered to be toxin-resistant.

17. The fusion protein according to any one of claims 1 to 16, wherein
the first linker L1 has an amino acid sequence shown in any one of SEQ ID NOs: 10, 11, 15, 16, 18 to 20, 31, and 43, or has an amino acid sequence with at least 80%, 85%, 90%, 95%, or 99% sequence identity to any one of SEQ ID NOs: 10, 11, 15, 16, 18 to 20, 31, and 43.

18. An isolated nucleic acid, wherein
the nucleic acid encodes the fusion protein according to any one of claims 1 to 17.

19. A vector, wherein the vector comprises the nucleic acid according to claim 18.

20. A host cell, comprising:
the nucleic acid according to claim 18 or the vector according to claim 19;
preferably, the host cell is a yeast cell;
further, the yeast cell is *Saccharomyces cerevisiae*, *Kluyveromyces*, or a combination thereof; in another preferred example, the *Kluyveromyces* is *Kluyveromyces lactis, Kluyveromyces marxianus*, *Kluyveromyces dobzhanskii*, or a combination thereof.

21. An *in vitro* cell-free protein synthesis system, wherein
an mRNA or DNA encoding the fusion protein according to any one of claims 1 to 17 is used as a template to synthesize the fusion protein.

22. The *in vitro* cell-free protein synthesis system according to claim 21, wherein the *in vitro* cell-free protein synthesis system comprises any one or more of the following:
(1) the mRNA or DNA template;
(2) a cell extract, wherein the cell extract is a yeast cell extract; further, the cell extract is derived from a strain selected from the group consisting of *Saccharomyces cerevisiae*, *Pichia pastoris*, and *Kluyveromyces,* or a combination thereof; preferably, the yeast cell extract is derived from *Kluyveromyces,* more preferably *Kluyveromyces lactis*;
(3) any one or more of the following components:
an amino acid mixture, dNTP, RNA polymerase, DNA polymerase, an energy supply system, polyethylene glycol, and an aqueous solvent.

23. The *in vitro* cell-free protein synthesis system according to claim 22, wherein
the strain is genetically engineered to be toxin-resistant; preferably, the strain exhibits reduced expression or activity of the DPH gene in the cell and/or a mutation in the amino acid sequence of eEF2; preferably, the expression level of the DPH gene is 10% or less, preferably 5% or less, and more preferably 2% or less; and/or the reduction in expression or activity of the DPH gene satisfies the following condition: the ratio of A1/A0 is 30% or less, preferably 10% or less, more preferably 5% or less, even more preferably 2% or less, and most preferably 0 to 2%, wherein A1 represents the expression or activity of the DPH gene in the genetically engineered cell, and A0 represents the expression or activity of the wild-type DPH gene.

24. An *in vitro* cell-free synthesis method for the fusion protein according to any one of claims 1 to 17, comprising:
in a reaction system comprising the *in vitro* cell-free protein synthesis system according to any one of claims 21 to 23, performing an *in vitro* synthesis reaction using an mRNA or DNA encoding the fusion protein according to any one of claims 1 to 17 as a template to obtain the fusion protein; preferably, the DNA template and other components involved in the *in vitro* synthesis reaction have a volume ratio of 1:10 to 1:50, or 1:20 to 1:40, or 1:25 to 1:35, or 1:30.

25. The *in vitro* cell-free synthesis method according to claim 24, wherein
the *in vitro* cell-free protein synthesis system comprises a yeast cell extract, an amino acid mixture, dNTP, RNA polymerase, DNA polymerase, an energy supply system, polyethylene glycol, and an aqueous solvent;
further:
the yeast cell extract is derived from a strain selected from the group consisting of *Saccharomyces cerevisiae*, *Pichia pastoris*, and *Kluyveromyces,* or a combination thereof; preferably, the yeast cell extract is derived from *Kluyveromyces,* more preferably *Kluyveromyces lactis*;
further, the strain is genetically engineered to be toxin-resistant.

26. The *in vitro* cell-free synthesis method according to claim 25, wherein
the yeast cell extract accounts for 50 to 80% of the total volume of the *in vitro* cell-free protein synthesis system.

27. Use of the nucleic acid according to claim 18, the vector according to claim 19, the host cell according to claim 20, or the *in vitro* cell-free protein synthesis system according to any one of claims 21 to 23 in the manufacture of the fusion protein according to any one of claims 1 to 18.

28. Use of the fusion protein according to any one of claims 1 to 17 in disease treatment, or a medicament comprising the fusion protein according to any one of claims 1 to 17 as an active ingredient for treating a disease, preferably for use in anti-tumor therapy, anti-transplant rejection, and treatment of an autoimmune disease; more preferably, the disease treatment is a treatment targeting any one or more of HER2 and CD22.

29. Use of the nucleic acid according to claim 18, the vector according to claim 19, the host cell according to claim 20, or the fusion protein according to any one of claims 1 to 17 in the manufacture of a medicament for the use according to claim 28.
